# EUROPEAN PATENT APPLICATION

(11) **EP 2 072 509 A1**
(43) Date of publication of application: **24.06.2009**
(21) Application number: 07076098.8
(22) Date of filing: 18.12.2007
(51) Int. Cl.: C07D 401/04, C07D 215/227, A61K 31/4709, A61K 31/4704, A61P 29/00

(54) **1-Aryl-1H-quinoline-2-ones: process for their production and their use as anti-inflammatory agents**

(71) Applicant: Bayer Schering Pharma Aktiengesellschaft, 13353 Berlin (DE); AstraZeneca AB, 151 85 Södertälje (SE)
(72) Inventor: Rehwinkel, Hartmut, Dr., 10961 Berlin (DE); Berger, Markus, Dr., 13347 Berlin (DE); Schäcke, Heike, Dr., 10115 Berlin (DE)

(57) **Abstract**

The present invention relates to the compounds of formula I, 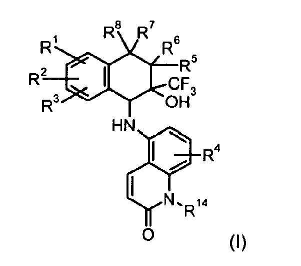 processes for their production and their use as anti-inflammatory agents.

## Description

The present invention relates to compounds of formula I, a process for their production and their use as anti-inflammatory agents.

From the prior art of WO 2005/034939 , anti-inflammatory agents of the following general formula 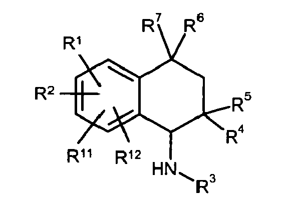 are known. In the experiment, these compounds show dissociation of action between anti-inflammatory and undesirable metabolic actions and are superior to the previously described nonsteroidal glucocorticoids or exhibit at least just as good an action.

It was therefore the object of this invention to make compounds available whose selectivity towards the glucocorticoid receptor (GR) is improved compared to the other steroid receptors.

This object has been achieved by the compounds according to the claims.

This invention therefore relates to compounds of general formula I 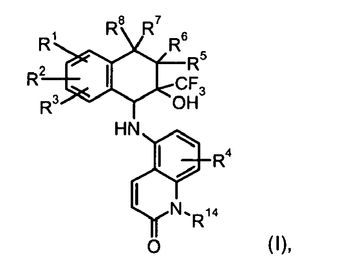 in which
- R¹, R², R³: independently of one another, are selected from the group consisting of hydrogen, halogen, cyano, nitro, hydroxy, (C₁-C₅)-alkyl, (C₁-C₅)-halo-alkyl, (C₁-C₅)alkoxy, (C₁-C₅)halo-alkoxy and COOR¹³, and in which two vicinal substituents together may form a group that is selected from the groups -O-(CH₂)ₚ-O-, -O-(CH₂)ₚ-CH₂-, -O-CH=CH-, -(CH₂)ₚ₊₂-, -NH-(CH₂)ₚ₊₁-, -N(C₁-C₃-alkyl)-(CH₂)ₚ₊₁-, and -NH-N=CH-,
in which p = 1 or 2, and in which R¹³ means hydrogen or C₁-C₅-alkyl,
- R⁴: is selected from the group consisting of hydrogen, halogen, cyano, nitro hydroxy, (C₁-C₅)-alkyl, (C₁-C₅)-halo-alkyl, (C₁-C₅)alkoxy, (C₁-C₅)halo-alkoxy and COOR¹³, in which R¹³ means hydrogen or C₁-C₅-alkyl,
- R⁵, R⁶: independently of one another, are selected from the group consisting of hydrogen or (C₁-C₅)-alkyl or (C₁-C₅)-halo-alkyl,
- R⁷, R⁸: independently of one another, are selected from the group consisting of hydrogen or (C₁-C₅)alkyl, (C₁-C₅)-halo-alkyl, or in which R⁵ and R⁶ together, or R⁵ and R⁷ together, or R⁵ and R⁸ together, or R⁶ and R⁷ together, or R⁶ and R⁸ together, or R⁷ and R⁸ together may form a C₃-C₈ cycloalkyl ring,
- R⁹, R¹⁰, R¹¹: independently of one another, are selected from the group consisting of hydrogen, halogen, cyano, nitro, hydroxy, (C₁-C₅)-alkyl, (C₁-C₅)-halo-alkyl, (C₁-C₅)alkoxy, (C₁-C₅)halo-alkoxy and COOR¹³, in which R¹³ means hydrogen or C₁-C₅-alkyl,
- R¹⁴: is a group 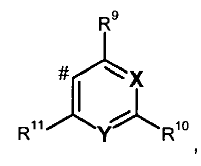 in which
# denotes the point of attachment of the R¹⁴ group via a single bond,
- X, Y: independently of one another are nitrogen, or a group C- R¹² in which R¹² is selected from the group consisting of hydrogen, halogen, cyano, nitro, hydroxy, (C₁-C₅)-alkyl, (C₁-C₅)-halo-alkyl, (C₁-C₅)alkoxy, (C₁-C₅)halo-alkoxy and COOR¹³, in which R¹³ means hydrogen or C₁-C₅-alkyl.

### One aspect of the invention are compounds of general formula I, in which

- R¹, R², R³: independently of one another, are selected from the group consisting of hydrogen, halogen, hydroxy, (C₁-C₅)-alkyl, (C₁-C₅)alkoxy,
- R⁴: is selected from the group consisting of hydrogen, halogen, hydroxy, or (C₁-C₅)-alkyl, (C₁-C₅)alkoxy,
- R⁵, R⁶: independently of one another, are selected from the group consisting of hydrogen or (C₁-C₅)-alkyl,
- R⁷, R⁸: independently of one another, are selected from the group consisting of hydrogen or (C₁-C₅)-alkyl, or in which R⁵ and R⁶ together, or R⁵ and R⁷ together, or R⁵ and R⁸ together, or R⁶ and R⁷ together, or R⁶ and R⁸ together, or R⁷ and R⁸ together may form a C₃-C₈ cycloalkyl ring,
- R⁹, R¹⁰, R¹¹: independently of one another, are selected from the group consisting of hydrogen, halogen, cyano, hydroxy, (C₁-C₅)-alkyl, (C₁-C₅)-halo-alkyl,
- R¹⁴: is a group 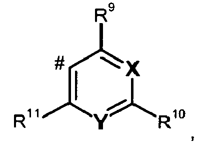 in which
# denotes the point of attachment of the R¹⁴ group via a single bond,
- X, Y: independently of one another are nitrogen, or a group C- R¹² in which R¹² is selected from the group consisting of hydrogen, halogen, cyano, nitro, hydroxy, (C₁-C₅)-alkyl, (C₁-C₅)-halo-alkyl, (C₁-C₅)alkoxy, (C₁-C₅)halo-alkoxy and COOR¹³, in which R¹³ means hydrogen or C₁-C₅-alkyl.

### Another aspect of the invention are compounds of general formula I, in which

- R¹, R², R³: independently of one another, are selected from the group consisting of hydrogen, chloro, hydroxy, (C₁-C₄)-alkyl, methoxy,
- R⁴: is selected from the group consisting of hydrogen or fluoro,
- R⁵, R⁶: independently of one another, are selected from the group consisting of hydrogen or methyl,
- R⁷, R⁸: independently of one another, are selected from the group consisting of hydrogen or methyl, or in which R⁵ and R⁶ together, or R⁵ and R⁷ together, or R⁵ and R⁸ together, or R⁶ and R⁷ together, or R⁶ and R⁸ together, or R⁷ and R⁸ together may form a C₃-C₈ cycloalkyl ring,
- R⁹, R¹⁰, R¹¹: independently of one another, are selected from the group consisting of hydrogen, fluoro, chloro, cyano, hydroxy, methyl or trifluoromethyl,
- R¹⁴: is a group 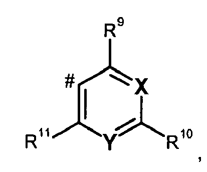 in which
# denotes the point of attachment of the R¹⁴ group via a single bond,
- X, Y: independently of one another are nitrogen or a group C- R¹²,
in which R¹² is selected from the group consisting of hydrogen, fluoro, chloro, cyano, hydroxyl or methyl.

Unless otherwise notified the term "alkyl" refers to straight or branched saturated hydrocarbon with 1 to 5, preferred 1 to 4, more preferred 1 to 3 carbon atoms. For example: methyl-, ethyl-, propyl-, butyl-, pentyl-, -, iso-propyl-, iso-butyl-, sec-butyl, tert-butyl-, isopentyl-, 2-methylbutyl-, 1-methylbutyl-, 1-ethylpropyl-, 1,2-dimethyl-propyl, neo-pentyl-, 1,1-dimethylpropyl. Most preferred are methyl-, ethyl-, propyl- or isopropyl.

Unless otherwise notified the term "halo-alkyl" refers to straight or branched alkyl derivatives in which at least one hydrogen is substituted by a halogen atom, preferably by a fluoro atom. For example: Trifluoromethyl, 2,2,2-trifluoroethyl-, pentafluoro-ethyl-, 5,5,5,4,4-pentafluoropentyl- or 5,5,5,4,4,3,3-heptafluoropentyl-.

Preferred are perfluorinated alkyl residues like trifluoromethyl or pentafluoroethyl.

For example, the term halopropyl comprises perfluoro-*ⁿ*-propyl and perfluoro*^{iso}*-propyl, as well as 1-chloro propyl, the term haloethyl comprises 1-fluoroethyl, 2,2,2- trifluoroethyl, 1-chloroethyl, 2-chloroethyl and 2-bromoethyl.

Unless otherwise notified the term "halo-alkoxy" refers to straight or branched saturated alkoxy derivatives in which at least one hydrogen is substituted by a halogen atom, preferably by a fluoro atom. For example: 2,2,2-trifluoroethoxy.

Unless otherwise notified the term "cycloalkyl" refers to a monocyclic, saturated, divalent hydrocarbon with 1 to 10, preferred 1 to 8, more preferred 1 to 6 carbon atoms

For example: Cyclopropyl, cyclobutyl, cyclopentyl, cyclohexyl or cycloheptyl. Preferred is cyclopropyl-, cylopentyl- or cyclohexyl.

Unless otherwise notified the term "alkoxy" refers to straight or branched saturated alkylether residue of formula -O-Alkyl with 1 to 5, preferred 1 to 4, more preferred 1 to 3 carbon atoms. For example: Methoxy, Ethoxy, n-Propoxy, Isopropoxy, tert.-Butoxy, and n-Pentoxy. A methoxy or ethoxy group is preferred.

The designation halogen atom or halogen means a fluorine, chlorine, bromine or iodine atom. Preferred is a fluorine, chlorine or bromine atom. More preferred is a fluorine or chlorine atom.

Compounds of general formula I, in which at least one of the groups R¹-R³ is selected from the group consisting of Fluoro, Chloro, Hydroxy, C₁-C₃-Alkyl, C₁-C₃-Alkoxy are a preferred subject of the invention.

Preferred groups R⁴ are Hydrogen, Fluoro, Chloro, C₁-C₃-Alkyl and C₁-C₃-Alkoxy.

Preferred groups R⁵ and R⁶ are Hydrogen and C₁-C₃-Alkyl.

Preferred groups R⁷ and R⁸ are hydrogen and the other is methyl or ethyl or in which both of R⁷ and R⁸ are methyl or ethyl.

Preferred groups R⁹, R¹⁰, R¹¹ are independently of one another selected from the group consisting of hydrogen, halogen, cyano, hydroxy, (C₁-C₅)-alkyl.

Preferred groups X, Y are independently of one another are nitrogen, or a group C- R¹² in which R¹² is selected from the group consisting of hydrogen, halogen, cyano, hydroxy, (C₁-C₅)-alkyl, (C₁-C₅)-halo-alkyl, and

R¹⁴ is a group 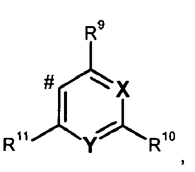 in which # denotes the point of attachment of the R¹⁴ group via a single bond.

In addition, the invention relates to the use of the compounds of general formula I for the production of pharmaceutical agents as well as their use for the production of pharmaceutical agents for treating inflammatory diseases.

Due to the presence of asymmetry centers the compounds of general formula I according to the invention may have several stereoisomers. Subjects of this invention are all possible enantiomers and diastereomers, both as racemates and in enantiomer-pure form.

The compounds according to the invention can also be present in the form of salts with physiologically compatible anions, for example in the form of hydrochlorides, sulfates, nitrates, phosphates, pivalates, maleates, fumarates, tartrates, benzoates, mesylates, citrates or succinates.

The process for the production of the compounds of WO2005/034939, WO2006/027236, WO2006/066950 and WO2006/100100 can also be used for the production of the compounds according to the invention.
A) An quinoline of general formula (II), in which , R¹, R², R³, R⁴, R⁵, R⁶, R⁷ and R⁸ have the meanings that are indicated for formula (I), is converted with an arylboronic acid of the general formula (III), in which under R¹⁴ has the meaning that is indicated for formula (I), under catalysis of copper or palladium (P. Lam et al. Syn. Lett. 2000; 5, 674 - 76; H. Sano et al, Biorg. Med. Chem. 2005, 13, 3079-91) to compounds of the general formula (I) according to the invention. 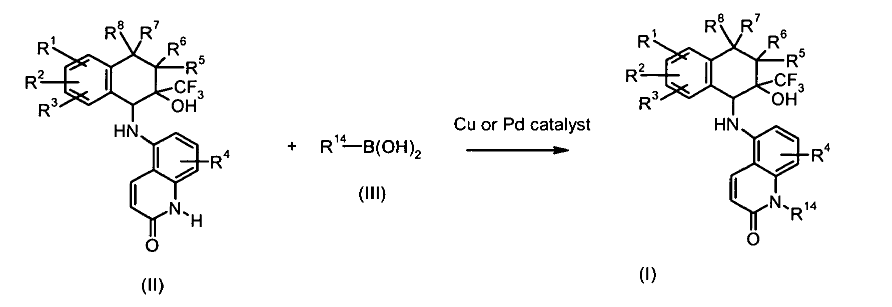
B) Title compound (I) can also be synthesized with by imine formation of an aldehyde of general formula (IV), in which R¹, R², R³, R⁵, R⁶, R⁷ and R⁸ have the meanings that are indicated for formula (I), and an aminoquinoline-2-one of general formula (V), in which R⁴ and R¹⁴ are defined as described in formula I according to the methods that are known to one skilled in the art, whereby e.g. acetic acid or titanium tetra alcoholates are considered as acidic catalysts for the imine formation.

The so formed imine can be treated with inorganic acids or organic acids or Lewis acids at temperatures between (-50°C) and (+20°C) for the formation of the stereoisomer of formula I according to the invention.

The reaction can be carried out in halogenated solvents. Preferred is dichloro-methane. Suitable acids are inorganic acids (e. g. hydrochloric acid, sulfuric acid) or organic acids (e.g. acetic acid) or the common Lewis acids (e.g. aluminum trichloride, boron tribromide, titanium tetrachloride). Preferred are boron tribromide or titanium tetrachloride.

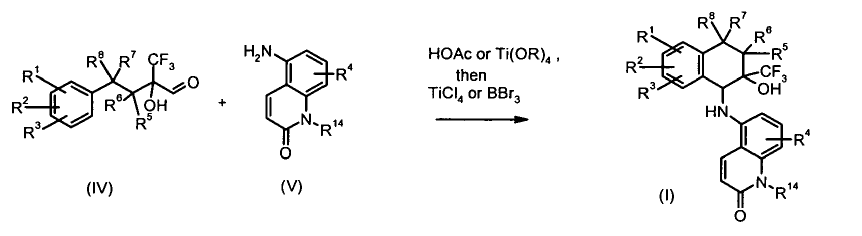

If the compounds according to the invention are present as racemic mixtures, they can be separated into pure, optically active forms according to the methods of racemate separation that are familiar to one skilled in the art. For example, the racemic mixtures can be separated by chromatography on an even optically active carrier material (CHIRALPAK AD^{®}) into the pure isomers. It is also possible to esterify the free hydroxy group in a racemic compound of general formula I with an optically active acid and to separate the diastereoisomeric esters that are obtained by fractionated crystallization or by chromatography, and to saponify the separated esters in each case to the optically pure isomers. As an optically active acid, for example, mandelic acid, camphorsulfonic acid or tartaric acid can be used.

The binding of the substances to the glucocorticoid receptor (GR) and other steroid hormone receptors (mineral corticoid receptor (MR), progesterone receptor (PR) and androgen receptor (AR)) is examined with the aid of recombinantly produced receptors. Cytosol preparations of Sf9 cells, which had been infected with recombinant baculoviruses, which code for the GR, are used for the binding studies. In comparison to reference substance [³H]-dexamethasone, the substances show a high to very high affinity to GR. Example 2 thus shows, e.g., the following profile: IC₅₀(GR) = 32 nmol; IC₅₀(MR), IC₅₀(PR), IC₅₀(AR), IC₅₀(ER) > 1 µmol.

As an essential, molecular mechanism for the anti-inflammatory action of glucocorticoids, the GR-mediated inhibition of the transcription of cytokines, adhesion molecules, enzymes and other pro-inflammatory factors is considered. This inhibition is produced by an interaction of the GR with other transcription factors, e.g., AP-1 and NF-kappa-B (for a survey, see Cato, A. C. B., and Wade, E., BioEssays 18, 371-378, 1996).

The compounds of general formula I according to the invention inhibit the secretion of cytokine IL-8 into the human monocyte cell line THP-1 that is triggered by lipopolysaccharide (LPS). The concentration of the cytokines was determined in the supernatant by means of commercially available ELISA kits. The compound of Example 1 showed an inhibition IC₅₀(IL8) = 30 nM (84,1 % eff. relative to dexamethasone).

The anti-inflammatory action of the compounds of general formula I was tested in the animal experiment by tests in the croton oil-induced inflammation in rats and mice (J. Exp. Med. (1995), 182, 99-108). To this end, croton oil in ethanolic solution was applied topically to the animals' ears. The test substances were also applied topically or systemically at the same time or two hours before the croton oil. After 16-24 hours, the ear weight was measured as a yardstick for inflammatory edema, the peroxidase activity as a yardstick for the invasions of granulocytes, and the elastase activity as a yardstick for the invasion of neutrophilic granulocytes. In this test, the compounds of general formula I inhibit the three above-mentioned inflammation parameters both after topical administration and after systemic administration.

One of the most frequent undesirable actions of a glucocorticoid therapy is the so-called "steroid diabetes" [cf., Hatz, H. J., Glucocorticoide: Immunologische Grundlagen, Pharmakologie und Therapierichtlinien [Glucocorticoids: Immunological Bases, Pharmacology and Therapy Guidelines], Wissenschaftliche Verlagsgesellschaft mbH, Stuttgart, 1998]. The reason for this is the stimulation of gluconeogenesis in the liver by induction of the enzymes responsible in this respect and by free amino acids, which are produced from the degradation of proteins (catabolic action of glucocorticoids). A key enzyme of the catabolic metabolism in the liver is tyrosinamino transferase (TAT). The activity of this enzyme can be determined from liver homogenates by photometry and represents a good measurement of the undesirable metabolic actions of glucocorticoids. To measure the TAT induction, the animals are sacrificed 8 hours after the test substances are administered, the livers are removed, and the TAT activity is measured in the homogenate. In this test, at doses wherein they have an anti-inflammatory action, the compounds of general formula I induce little or no tyrosinamino transferase.

Because of their anti-inflammatory and, in addition, anti-allergic, immuno-suppressive and antiproliferative action, the compounds of general formula I according to the invention can be used as medications for treatment or prophylaxis of the following pathologic conditions in mammals and humans: In this case, the term "DISEASE" stands for the following indications:
(i) Lung diseases, which coincide with inflammatory, allergic and/or proliferative processes:
   - Chronic, obstructive lung diseases of any origin, primarily bronchial asthma
   - Bronchitis of different origins
   - Adult respiratory distress syndrome (ARDS), acute respiratory distress syndrome
   - Bronchiectases
   - All forms of restrictive lung diseases, primarily allergic alveolitis,
   - All forms of pulmonary edema, primarily toxic pulmonary edema; e.g., radiogenic pneumonitis
   - Sarcoidoses and granulomatoses, especially Boeck's disease
(ii) Rheumatic diseases/autoimmune diseases/joint diseases, which coincide with inflammatory, allergic and/or proliferative processes:
   - All forms of rheumatic diseases, especially rheumatoid arthritis, acute rheumatic fever, polymyalgia rheumatica, Behçet's disease
   - Reactive arthritis
   - Inflammatory soft-tissue diseases of other origins
   - Arthritic symptoms in the case of degenerative joint diseases (arthroses)
   - Traumatic arthritides
   - Vitiligo
   - Collagenoses of any origin, e.g., systemic lupus erythematodes, sclerodermia, polymyositis, dermatomyositis, Sjögren's syndrome, Still's syndrome, Felty's syndrome
   - Sarcoidoses and granulomatoses
   - Soft-tissue rheumatism
(iii) Allergies or pseudoallergic diseases, which coincide with inflammatory and/or proliferative processes:
   - All forms of allergic reactions, e.g., Quincke's edema, hay fever, insect bites, allergic reactions to pharmaceutical agents, blood derivatives, contrast media, etc., anaphylactic shock, urticaria, allergic and irritative contact dermatitis, allergic vascular diseases
   - Allergic vasculitis
(iv) Vascular inflammations (vasculitides)
   - Panarteritis nodosa, temporal arteritis, erythema nodosum
   - Polyarteris nodosa
   - Wegner's granulomatosis
   - Giant-cell arteritis
(v) Dermatological diseases, which coincide with inflammatory, allergic and/or proliferative processes:
   - Atopic dermatitis (primarily in children)
   - All forms of eczema, such as, e.g., atopic eczema (primarily in children)
   - Rashes of any origin or dermatoses
   - Psoriasis and parapsoriasis groups
   - Pityriasis rubra pilaris
   - Erythematous diseases, triggered by different noxae, e.g., radiation, chemicals, burns, etc.
   - Bullous dermatoses, such as, e.g., autoimmune pemphigus vulgaris, bullous pemphigoid
   - Diseases of the lichenoid group,
   - Pruritis (e.g., of allergic origin)
   - Seborrheal eczema
   - Rosacea group
   - Erythema exudativum multiforme
   - Balanitis
   - Vulvitis
   - Manifestation of vascular diseases
   - Hair loss such as alopecia areata
   - Cutaneous lymphoma
(vi) Kidney diseases, which coincide with inflammatory, allergic and/or proliferative processes:
   - Nephrotic syndrome
   - All nephritides, e.g., glomerulonephritis
(vii) Liver diseases, which coincide with inflammatory, allergic and/or proliferative processes:
   - Acute liver cell decomposition
   - Acute hepatitis of different origins, e.g., viral, toxic, pharmaceutical agent-induced
   - Chronic aggressive hepatitis and/or chronic intermittent hepatitis
(viii) Gastrointestinal diseases, which coincide with inflammatory, allergic and/or proliferative processes:
   - Regional enteritis (Crohn's disease)
   - Colitis ulcerosa
   - Gastritis
   - Reflux esophagitis
   - Ulcerative colitis of other origins, e.g., native sprue
(ix) Proctologic diseases, which coincide with inflammatory, allergic and/or proliferative processes:
   - Anal eczema
   - Fissures
   - Hemorrhoids
   - Idiopathic proctitis
(x) Eye diseases, which coincide with inflammatory, allergic and/or proliferative processes:
   - Allergic keratitis, uveitis, iritis
   - Conjunctivitis
   - Blepharitis
   - Optic neuritis
   - Chorioiditis
   - Sympathetic ophthalmia
(xi) Diseases of the ear-nose-throat area, which coincide with inflammatory, allergic and/or proliferative processes:
   - Allergic rhinitis, hay fever
   - Otitis externa, e.g., caused by contact dermatitis, infection, etc.
   - Otitis media
(xii) Neurological diseases, which coincide with inflammatory, allergic and/or proliferative processes:
   - Cerebral edema, primarily tumor-induced cerebral edema
   - Multiple sclerosis
   - Acute encephalomyelitis
   - Meningitis
   - Various forms of convulsions, e.g., infantile nodding spasms
   - Acute spinal cord injury
   - Stroke
(xiii) Blood diseases, which coincide with inflammatory, allergic and/or proliferative processes, such as, e.g.: M. Hodgkins or Non-Hodgkins lymphomas, thrombocythemias, erythrocytoses
   - Acquired hemolytic anemia
   - Idiopathic thrombocytopenia
(xiv) Tumor diseases, which coincide with inflammatory, allergic and/or proliferative processes, such as, e.g.: carcinomas or sarcomas
   - Acute lymphatic leukemia
   - Malignant lymphoma
   - Lymphogranulomatoses
   - Lymphosarcoma
   - Extensive metastases, mainly in breast, bronchial and prostate cancers
(xv) Endocrine diseases, which coincide with inflammatory, allergic and/or proliferative processes, such as, e.g.:
   - Endocrine orbitopathy
   - Thyreotoxic crisis
   - De Quervain's thyroiditis
   - Hashimoto's thyroiditis
   - Basedow's disease
   - Granulomatous thyroiditis
   - Lymphadenoid goiter
(xvi) Organ and tissue transplants, graft-versus-host disease
(xvii) Severe shock conditions, e.g., anaphylactic shock, systemic inflammatory response syndrome (SIRS)
(xviii) Substitution therapy in:
   - Innate primary suprarenal insufficiency, e.g., congenital adrenogenital syndrome
   - Acquired primary suprarenal insufficiency, e.g., Addison's disease, autoimmune adrenalitis, meta-infective tumors, metastases, etc.
   - Innate secondary suprarenal insufficiency, e.g., congenital hypopituitarism
   - Acquired secondary suprarenal insufficiency, e.g., meta-infective tumors, etc.
(xix) Emesis, which coincide with inflammatory, allergic and/or proliferative processes:
   - e.g., in combination with a 5-HT3 antagonist in cytostatic-agent-induced vomiting
(xx) Pains of inflammatory origins, e.g., lumbago
(xxi) Other different stages of disease including diabetes type I (insulin-dependent diabetes), osteoarthritis, Guillain-Barré syndrome, restenoses after percutaneous transluminal angioplasty, Alzheimer's disease, acute and chronic pain, arteriosclerosis, reperfusion injury, congestive heart failure, myocardial infarction, thermal injury, multiple organ injury secondary to trauma, acute purulent meningitis, necrotizing enterocolitis and syndromes associated with hemodialysis, leukopheresis, and granulocyte transfusion.

Moreover, the compounds of general formula I according to the invention can be used for treatment and prophylaxis of additional pathologic conditions that are not mentioned above, for which synthetic glucocorticoids are now used (see in this respect Hatz, H. J., Glucocorticoide: Immunologische Grundlagen, Pharmakologie und Therapierichtlinien, Wissenschaftliche Verlagsgesellschaft mbH, Stuttgart, 1998).

All previously mentioned indications (i) to (xxi) are described in more detail in Hatz, H. J., Glucocorticoide: Immunologische Grundlagen, Pharmakologie und Therapierichtlinien, Wissenschaftliche Verlagsgesellschaft mbH, Stuttgart, 1998.

For the therapeutic actions in the above-mentioned pathologic conditions, the suitable dose varies and depends on, for example, the active strength of the compound of general formula I, the host, the type of administration, and the type and severity of the conditions that are to be treated, as well as the use as a prophylactic agent or therapeutic agent.

In addition, the invention provides:
(i) The use of one of the compounds of formula I according to the invention or mixture thereof for the production of a medication for treating a DISEASE;
(ii) A process for treating a DISEASE, said process comprises an administration of an amount of the compound according to the invention, in which the amount suppresses the disease and in which the amount of compound is given to a patient who requires such a medication;
(iii) A pharmaceutical composition for treating a DISEASE, said treatment comprises one of the compounds according to the invention or mixture thereof and at least one pharmaceutical adjuvant and/or vehicle.

In general, satisfactory results can be expected in animals when the daily doses comprise a range of 1 µg to 100,000 µg of the compound according to the invention per kg of body weight. In the case of larger mammals, for example the human, a recommended daily dose lies in the range of 1 µg to 100,000 µg per kg of body weight. Preferred is a dose of 10 to 30,000 µg per kg of body weight, and more preferred is a dose of 10 to 10,000 µg per kg of body weight. For example, this dose is suitably administered several times daily. For treating acute shock (e.g., anaphylactic shock), individual doses can be given that are significantly above the above-mentioned doses.

The formulation of the pharmaceutical preparations based on the new compounds is carried out in a way that is known in the art by the active ingredient being processed with the vehicles that are commonly used in galenicals, fillers, substances that influence decomposition, binding agents, moisturizers, lubricants, absorbents, diluents, flavoring correctives, coloring agents, etc., and converted into the desired form of administration. In this case, reference is made to Remington's Pharmaceutical Science, 15th Edition, Mack Publishing Company, East Pennsylvania (1980).

For oral administration, especially tablets, coated tablets, capsules, pills, powders, granulates, lozenges, suspensions, emulsions or solutions are suitable.

For parenteral administration, injection and infusion preparations are possible.

For intra-articular injection, correspondingly prepared crystal suspensions can be used.

For intramuscular injection, aqueous and oily injection solutions or suspensions and corresponding depot preparations can be used.

For rectal administration, the new compounds can be used in the form of suppositories, capsules, solutions (e.g., in the form of enemas) and ointments both for systemic and for local treatment.

For pulmonary administration of the new compounds, the latter can be used in the form of aerosols and inhalants.

For local application to eyes, outer ear channels, middle ears, nasal cavities, and paranasal sinuses, the new compounds can be used as drops, ointments and tinctures in corresponding pharmaceutical preparations.

For topical application, formulations in gels, ointments, fatty ointments, creams, pastes, powders, milk and tinctures are possible. The dosage of the compounds of general formula I should be 0.01%-20% in these preparations to achieve a sufficient pharmacological action.

The invention also comprises the compounds of general formula I according to the invention as therapeutic active ingredients.

In addition, the compounds of general formula I according to the invention are part of the invention as therapeutic active ingredients together with pharmaceutically compatible and acceptable adjuvants and vehicles.

The invention also comprises a pharmaceutical composition that contains one of the pharmaceutically active compounds according to the invention or mixtures thereof or a pharmaceutically compatible salt thereof and a pharmaceutically compatible salt or pharmaceutically compatible adjuvants and vehicles.

The compounds of general formula (I) according to the invention can optionally also be formulated and/or administered in combination with other active ingredients.

The invention therefore also relates to combination therapies or combined compositions, wherein a compound of general formula (I) or a pharmaceutically acceptable salt thereof, or a pharmaceutical composition that contains a compound of general formula (I) or a pharmaceutically acceptable salt thereof, is administered either simultaneously (optionally in the same composition) or in succession together with one or more pharmaceutical agents for treating one of the above-mentioned pathologic conditions. For example, for treatment of rheumatoid arthritis, osteoarthritis, COPD (chronic obstructive lung disease), asthma or allergic rhinitis, a compound of general formula (I) of this invention can be combined with one or more pharmaceutical agents for treating such a condition. When such a combination is administered by inhalation, the pharmaceutical agent that is to be combined can be selected from the following list:
- A PDE4 inhibitor including an inhibitor of the PDE4D isoform,
- A selective β.sub2.adrenoceptor agonist, such as, for example, metaproterenol, isoproterenol, isoprenaline, albuterol, salbutamol, formoterol, salmeterol, terbutaline, orcipresnaline, bitolterol mesylate, pirbuterol or indacaterol;
- A muscarine receptor antagonist (for example, an M1, M2 or M3 antagonist, such as, for example, a more selective M3 antagonist), such as, for example, ipratropium bromide, tiotropium bromide, oxitropium bromide, pirenzepine or telenzepine;
- A modulator of the chemokine receptor function (such as, for example, a CCR1 receptor antagonist);
- An inhibitor of the p38 kinase function,
- an inhibitor of matrix metalloproteases, most preferably targeting MMP-2, -9 or MMP-12,
- An inhibitor of neutrophil serine proteases, most preferably neutrophil elastase or proteinase 3

For another subject of this invention, such a combination with a compound of general formula (I) or a pharmaceutically acceptable salt thereof is used for treatment of COPD, asthma or allergic rhinitis and can be administered by inhalation or orally in combination with xanthine (such as, for example, aminophylline or thyeophylline), which also can be administered by inhalation or orally.

### Experimental Part:

The various aspects of the invention described in this application are illustrated by the following examples which are not meant to limit the invention in any way.

### Example 1

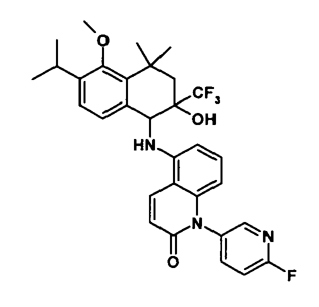

### 5-{[2-Hydroxy-6-isopropyl-5-methoxy-4,4-dimethyl-2-(trifluoromethyl)-1,2,3,4-tetrahydronaphthalene-1-yl]amino}-1-(6-fluoropyridine-3-yl)-1H-quinoline-2-one

5-{[2-Hydroxy-6-isopropyl-5-methoxy-4,4-dimethyl-2-(trifluoromethyl)-1,2,3,4-tetrahydronaphthalene-1-yl]amino}-1H-quinoline-2-one (46.5 mg, 0.098 mmol), described in WO2005/034939, 6-fluoropyridine-3-yl-boronic acid (27.6 mg, 0.2 mmol), pyridine (0.16 mL, 1.96 mmol), copper(II)acetate (19.6 mg, 0.098 mmol) and 4.2 mL dichloromethane are stirred for 22 hours at room temperature. The turquoise coloured solution is diluted with 50 mL ethyl acetate. The organic phase is washed with water (10 mL) and brine (10 mL). After drying over sodium sulfate, the solvent is evaporated and the residue purified by chromatography (LiChroprep NH₂) eluting with dichloromethane/ methanol. The title compound is isolated with a yield of 24.7% (13.8 mg). 28.3 mg (60.9%) of the starting material is recovered and is subjected again to the same reaction conditions. After three days of stirring and work-up as described above additional title compound (13.3 mg, 39.2%) is isolated.

¹H-NMR (300 MHz, CD₃OD): δ = 1.20 (6H), 1.49 (3H), 1.65 (3H), 2.08 (2H), 3.32 (1H, is largely under the solvent's signal), 3.77 (3H), 5.18 (1H), 5.90 (1H), 6.59 (1H), 6.68 (1H), 6.99-7.14 (2H), 7.25 (1H), 7.32 (1H), 7.92 (1H), 8.19 (1H), 8.30 (1H).

### Example 2

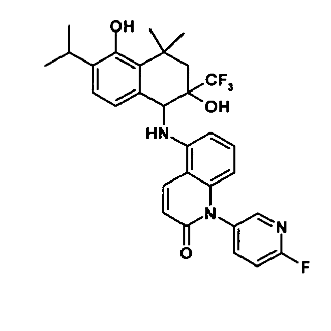

### 5-{[2,5-Dihydroxy-6-isopropyl-4,4-dimethyl-2-(trifluoromethyl)-1,2,3,4-tetrahydronaphthalene-1-yl]amino}-1-(6-fluoropyridine-3-yl)-1H-quinoline-2-one

5-{[2-Hydroxy-6-isopropyl-5-methoxy-4,4-dimethyl-2-(trifluoromethyl)-1,2,3,4-tetrahydronaphthalene-1-yl]amino}-1-(6-fluoropyridine-3-yl)-1H-quinoline-2-one (20.6 mg, 0.036 mmol) is given in 0.36 mL dichloromethane. The compound does not dissolve. At 0 °C 0.36 mL of an 1 M solution of boron tribromide in dichloromethane is added dropwise under a nitrogen atmosphere. The reaction mixture is stirred for three hours between -10 °C and 0 °C and then poured on a mixture of a saturated sodium bicarbonate solution and ice. After dilution with 30 mL ethyl acetate, the mixture is stirred vigorously for 20 minutes at room temperature. The organic phase is separated and washed with water (10 mL) and brine (10 mL) and dried (sodium sulfate). The solvent is evaporated and the residue purified by chromatography eluting with dichloromethane/ methanol. The desired product is obtained with a yield of 77.6% (15.6 mg).

¹H-NMR (300 MHz, CD₃OD): δ = 1.15-1.27 (6H), 1.61 (3H), 1.72 (3H), 2.02-2.20 (2H), 3.26 (1H), 5.19 (1H), 5.95 (1H), 6.63 (1H), 6.70 (1H), 6.87 (1H), 7.00 (1H), 7.30 (1H), 7.38 (1H), 7.99 (1H), 8.23 (1H), 8.34 (1H).

### Example 3

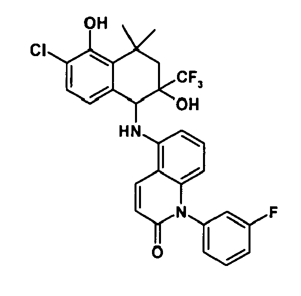

### 5-{[6-Chloro-2,5-dihydroxy-4,4-dimethyl-2-(trifluoromethyl)-1,2,3,4-tetrahydronaphthalene-1-yl]amino}-1-(3-fluorophenyl)-1H-quinoline-2-one

*5-{[4-(3-Chloro-2-methoxyphenyl)-2-hydroxy-4-methyl-2-trifluoromethyl)pentyliden]amino}1H-quinoline-2-one*

4-(3-Chloro-2-methoxyphenyl)-2-hydroxy-4-methyl-2-(trifluoromethyl)-pentanal (1.14 g, 3.51 mmol), described in WO2005/034939, which contains a certain amount of the deschloro compound as well, 5-amino-1H-quinoline-2-one (562.31 mg, 3.51 mmol) and 5.02 mL acetic acid are stirred for four days at room temperature. The solvent is evaporated three times with toluene. The residue is purified by column chromatography (Flashmaster) eluting with hexane/ ethyl acetate to yield 1.37 g (83.6%) of the title compound jointly with the corresponding deschloro derivative.

### 5-{[6-Chloro-2-hydroxy-5-methoxy-4,4-dimethyl-2-(trifluoromethyl)-1,2, 3, 4-tetrahydronaphthalene-1-y/]amino}-1H-quinoline-2-one

The mixture of 5-{[4-(3-Chloro-2-methoxyphenyl)-2-hydroxy-4-methyl-2-(trifluoromethyl)-pentyliden]amino}-1H-quinoline-2-one and the corresponding deschloro derivative (1.37 g, 2.93 mmol) is dissolved in 21.8 mL dichloromethane. Titanium tetrachloride (8.8 mL of an 1 M solution in dichloromethane) is added dropwise at 0 °C under a nitrogen atmosphere. After stirring for two hours between 0 and 2 °C, four hours between 2 and 15 °C and additional 16 hours at room temperature the reaction mixture is poured on a mixture of saturated sodium bicarbonate solution and ice. The mixture is diluted with 100 mL ethyl acetate and vigorously stirred for 20 minutes. The ethyl acetate phase is separated and the aqueous phase washed once with 50 mL ethyl acetate. The combined organic extracts are washed with water and brine. After drying the solvent is evaporated. The residue is purified by column chromatography eluting with dichloromethane/ methanol to give a mixture (1.02 g, 74.4%) of the title compound and the deschloro derivative.

### 5-{[6-Chloro-2-hydroxy-5-methoxy-4,4-dimethyl-2-(trifluoromethyl)-1,2,3,4-tetrahydronaphthalene-1-yl]amino}-1-(3-fluorophenyl)-1H-quinoline-2-one

The mixture of the 5-{[6-Chloro-2-hydroxy-5-methoxy-4,4-dimethyl-2-(trifluoromethyl)-1,2,3,4-tetrahydronaphthalene-1-yl]amino}-1H-quinoline-2-one and its deschloro derivative (160 mg, 0.34 mmol), 3-fluorophenyl boronic acid (95.9 mg, 0.69 mmol), pyridine (0.55 mL, 6.85 mmol), copper(II)acetate (68.4 mg, 0.34 mmol) and 14.6 mL dichloromethane are stirred for 28 hours at room temperature. The turquoise coloured solution is diluted with 150 mL ethylacetate. The organic phase is washed with water and brine. After drying over sodium sulfate, the solvent is evaporated and the residue purified by chromatography eluting with dichloromethane/ methanol. 133.4 mg (69.4%) of the title compound jointly with its deschloro derivative (strongly contaminated) are isolated.

### 5-{[6-Chloro-2,5-dihydroxy-4,4-dimethyl-2-(trifluoromethyl)-1,2,3,4-fetrahydronapha/ene-1-yl]amino}-1-(3-fluorophenyl)-1H-quinoline-2-one

The mixture of 5-{[6-Chloro-2-hydroxyl-5-methoxy-4,4-dimethyl-2-(trifluoromethyl)-1,2,3,4-tetrahydronaphthalene-1-yl]amino}-1-(3-fluorophenyl)-1H-quinoline-2-one and the deschloro derivative (130 mg, 0.23 mmol) is dissolved in 2.3 mL dichloromethane. At 0 °C 2.32 mL of an 1 M solution of boron tribromide in dichloromethane is added dropwise under nitrogen atmosphere. The reaction mixture is stirred for three hours between 0 °C and 15 °C. The reaction mixture is poured on a mixture of a saturated sodium bicarbonate solution and ice. After dilution with 100 mL ethyl acetate the mixture is stirred vigorously for 20 minutes at room temperature. The organic phase is washed with water and brine and dried (sodium sulfate ). The solvent is evaporated and the residue purified by chromatography (LiChroprep NH₂) eluting with dichloromethane. It is possible to separate the chloro and the deschloro compounds by chromatography at this stage. The title compound is obtained in pure form with a yield of 48.6% (61.1 mg). The deschloro compound is isolated with a yield of 6.4% (7.6 mg).

Title compound; ¹H-NMR (300 MHz, CD₃OD): δ = 1.55 (3H), 1.69 (3H), 2.00-2.19 (2H), 5.15 (1H), 5.92 (1H), 6.60 (1H), 6.65 (1H), 6.81 (1H), 7.05-7.19 (3H), 7.21 (1H), 7.32 (1H), 7.62 (1H), 8.27 (1H).

### Example 4

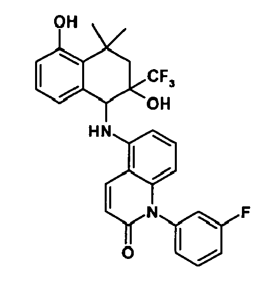

### 5-{[2,5-Dihydroxy-4,4-dimethyl-2-(trifluoromethyl)-1,2,3,4-tetrahydronaphthatene-1-yl] amino}-1-(3-fluorophenyl)-1H-quinoline-2-one

The compound is isolated as further product of Example 3. The synthesis is described in Example 3

Title compound; ¹H-NMR (400 MHz, CD₃OD): δ = 1.53 (3H), 1.68 (3H), 1.99-2.15 (2H), 5.15 (1H), 5.90 (1H), 6.55-6.70 (3H), 6.78 (1H), 6.91 (1H), 7.06-7.18 (2H), 7.22 (1H), 7.32 (1H), 7.62 (1H), 8.28 (1H).

### Example 5

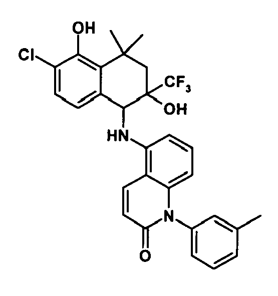

### 5-{[6-Chloro-2,5-dihydroxy-4,4-dimethyl-2-(trifluoromethyl)-1,2,3,4-tetrahydronaphthalene-1-yl]amino}-1-(3-methylphenyl)-1H-quinoline-2-one

*5-{[6-Chloro-2-hydroxy-5-methoxy-4,4-dimethyl-2-(trifluoromethyl)-1,2,3,4-tetrahydronaphthalene-1-yl]amino}-1-(4-fluorophenyl)-1H-quinoline-2-one*

5-{[6-Chloro-2-hydroxy-5-methoxy-4,4-dimethyl-2-(trifluoromethyl)-1,2,3,4-tetrahydronaphthalene-1-yl]amino}-1H-quinoline-2-one (160 mg, 0.34 mmol) jointly with the deschloro derivative, described in example 2, 3-methylphenyl boronic acid (93.2 mg, 0.69 mmol), pyridine (0.55mL, 6.85 mmol), copper(II)acetate (68.4 mg, 0.34 mmol) and 14.6 mL dichloromethane are stirred for 28 hours at room temperature. The turquoise coloured solution is diluted with 150 mL ethyl acetate. The organic phase is washed with water and brine. After drying over sodium sulfate, the solvent is evaporated and the residue purified by chromatography (LiChroprep NH₂) eluting with dichloromethane/ methanol yielding 37.4 mg (19.5%) of the title compound and the deschloro compound as a mixture (strongly contaminated).

*5-{[6-Chloro-2,5-dihydroxy-4,4-dimethyl-2-(trifluoromethyl)-1,2,3,4-tetrahydronaphthalene-1-yl]amino}-1-(3-methylphenyl)-1H-quinoline-2-one*

The mixture consisting of 5-{[6-Chloro-2-hydroxy-5-methoxy-4,4-dimethyl-2-(trifluoromethyl)-1,2,3,4-tetrahydronaphthalene-1-yl]amino}-1-(3-methylphenyl)-1H-quinoline-2-one and the deschloro derivative (35 mg, 0.063 mmol) is dissolved in 0.6 mL dichloromethane. At 0 °C 0.3 mL of an 1 M solution of boron tribromide in dichloromethane is added dropwise under nitrogen atmosphere. The reaction mixture is stirred for three hours between 0 °C and 15 °C. The reaction mixture is poured on a mixture of saturated sodium bicarbonate solution and ice. After dilution with 50 mL ethyl acetate the mixture is stirred vigorously for 20 minutes at room temperature. The organic phase is washed with water and brine and then dried (sodium sulfate ). The solvent is evaporated and the residue purified by chromatography eluting with dichloromethane. It is possible to separate the chloro and the deschloro compounds by chromatography at this stage. The title compound is obtained in pure form with a yield of 48.1 % (16.4 mg). The deschloro compound is isolated with a yield of 4.7% (1.5 mg).

Title compound; ¹H-NMR (300 MHz, CD₃OD): δ = 1.58 (3H), 1.70 (3H), 2.01-2.20 (2H), 2.45 (3H), 5.17 (1H), 5.92 (1H), 6.58-6.69 (2H), 6.82 (1H), 7.00-7.15 (3H), 7.20 (1H), 7.38 (1H), 7.50 (1H), 8.29 (1H).

### Example 6

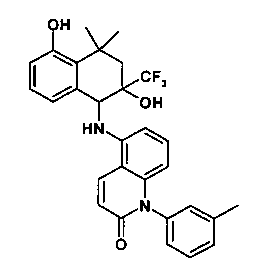

### 5-{[2,5-Dihydroxy-4,4-dimethyl-2-(trifluoromethyl)-1,2,3,4-tetrahydronaphthalene-1-yl]amino}-1-(3-methylphenyl)-1H-quinoline-2-one

The compound is isolated as further product of Example 5. The synthesis is described in Example 5.

Title compound; ¹H-NMR (300 MHz, CD₃OD): δ = 1.59 (3H), 1.70 (3H), 2.03-2.20 (2H), 2.49 (3H), 5.20 (1H), 5.95 (1H), 6.60-6.75 (3H), 6.82 (1H), 6.99 (1H), 7.07-7.19 (2H), 7.25 (1H), 7.41 (1H), 7.54 (1H), 8.32 (1H).

### Example 7

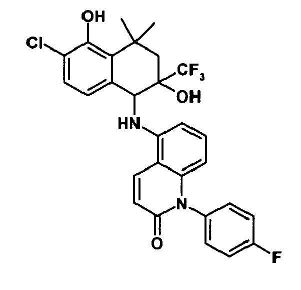

### 5-{[6-Chloro-2,5-dihydroxy-4,4-dimethyl-2-(trifluoromethyl)-1,2,3,4-tetrahydronaphthalene-1-yl]amino}-1-(4-fluorophenyl)-1H-quinoline-2-one

*5-{[6-Chloro-2-hydroxy-5-methoxy-4,4-dimethyl-2-(trifluoromethyl)-1,2,3,4-tetrahydronaphthalene-1-yl]amino}-1-(4-fluorophenyl)-1H-quinoline-2-one*

The mixture of 5-{[6-Chloro-2-hydroxy-5-methoxy-4,4-dimethyl-2-(trifluoromethyl)-1,2,3,4-tetrahydronaphthalene-1-yl]amino}-1H-quinoline-2-one (160 mg, 0.34 mmol) and the deschloro compound, described in example 3, 4-fluorophenyl boronic acid (95.9 mg, 0.69 mmol), pyridine (0.55mL, 6.85 mmol), copper(II)acetate (68.4 mg, 0.34 mmol) and 14.6 mL dichloromethane is stirred for 28 hours at room temperature. The turquoise coloured solution is diluted with 150 mL ethyl acetate. The organic phase is washed with water and brine. After drying over sodium sulfate, the solvent is evaporated and the residue purified by chromatography (LiChroprep) eluting with dichloromethane/ methanol yielding 58.6 mg (30.5%) of the title compound as a mixture with the deschloro compound.

*5-{[6-Chloro-2,5-dihydroxy-4,4-dimethyl-2-(trifluoromey*/*)-1,2,3,4-tetrahydronaphthalene-1-yl]amino}-1-(4-fluorophenyl)-1H-quinoline-2-one*

5-{[6-Chloro-2-hydroxy-5-methoxy-4,4-dimethyl-2-(trifluoromethyl)-1,2,3,4-tetrahydronaphthalene-1-yl]amino}-1-(4-fluorophenyl)-1H-quinoline-2-one (54 mg, 0.096 mmol) is dissolved in 0.9 mL dichloromethane. At 0 °C 0.96 mL of an 1 M solution of boron tribromide in dichloromethane is added dropwise under a nitrogen atmosphere. The reaction mixture is stirred for three hours between 0 °C and 15 °C and then poured on a mixture of saturated Sodium bicarbonate solution and ice. After dilution with 100 mL ethyl acetate the mixture is stirred vigorously for 20 minutes at room temperature. The organic phase is washed with water and brine and dried (sodium sulfate). The solvent is evaporated and the residue purified by chromatography (Lichroprep NH₂) eluting with dichloromethane/ methanol. It is possible to separate the chloro and the deschloro compounds at this stage. The title compound is isolated in pure form with a yield of 74.4 % (37.6 mg). The deschloro compound is isolated with a yield of 7.9% (3.9 mg).

Title compound; ¹H-NMR (400 MHz, CD₃OD): δ = 1.57 (3H), 1.69 (3H), 2.01-2.18 (2H), 5.18 (1H), 5.92 (1H), 6.56-6.68 (2H), 6.82 (1H), 7.10 (1H), 7.22 (1H), 7.24-7.39 (4H), 8.28 (1H).

### Example 8

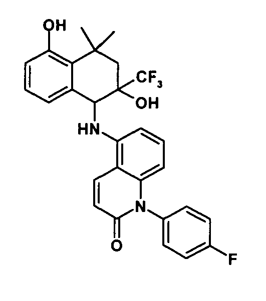

### 5-{[2,5-Dihydroxy-4,4-dimethyl-2-(trifluoromethyl)-1,2,3,4-tetrahydronaphthalene-1-yl]amino}-1-(4-fluorophenyl)-1H-quinoline-2-one

The compound is isolated as further product of Example 7. The synthesis is described in Example 7.

Title compound; ¹H-NMR (400 MHz, CD₃OD): δ = 1.53 (3H), 1.67 (3H), 1.99-2.13 (2H), 5.13 (1H), 5.90 (1H), 6.56-6.70 (3H), 6.78 (1H), 6.91 (1H), 7.21 (1H), 7.25-7.39 (4H), 8.28 (1H).

### Example 9

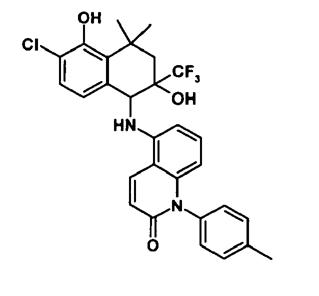

### 5-{[6-Chloro-2,5-dihydroxy-4,4-dimethyl-2-(trifluoromethyl)-1,2,3,4-tetrahydronaphthalene-1-yl]amino}-1-(4-methylphenyl)-1H-quinoline-2-one

*5-{[6-Chloro-2-hydroxy-5-methoxy-4,4-dimethyl-2-(trifluoromethyl)-1,2,3,4-tetrahydronaphthalene-1-yl]amino}-1-(4-methylphenyl)-1H-quinoline-2-one*

The mixture of 5-{[6-Chloro-2-hydroxy-5-methoxy-4,4-dimethyl-2-(trifluoromethyl)-1,2,3,4-tetrahydronaphthalene-1-yl]amino}-1H-quinoline-2-one and the corresponding deschloro derivative (290 mg, 0.62 mmol), described in example 2, 4-methylphenyl boronic acid (168.9 mg, 1.24 mmol), pyridine (1 mL, 12.42 mmol), copper(II)acetate (124.0 mg, 0.62 mmol) and 26.5 mL dichloromethane are stirred for 28 hours at room temperature. The turquoise coloured solution is diluted with 150 mL ethyl acetate. The organic phase is washed with water and brine. After drying over sodium sulfate, the solvent is evaporated and the residue purified by chromatography eluting with dichloromethane/ methanol yielding 44.2 mg (12.8%) of the title compound jointly with the deschloro derivative.

*5-{[6-Chloro-2,5-dihydroxy-4, 4-dimethyl-2-(trifluoromethyl)-1,2,3,4-tetrahydronaphthalene-1-yl]amino}-1-(4-methylphenyl)-1H-quinoline-2-one*

5-{[6-Chloro-2-hydroxy-5-methoxy-4,4-dimethyl-2-(trifluoromethyl)-1,2,3,4-tetrahydronaphthalene-1-yl]amino}-1-(4-methylphenyl)-1H-quinoline-2-one (40 mg, 0.072 mmol) is dissolved in 0.7 mL dichloromethane. At 0 °C is added dropwise 0.72 mL of an 1 M solution of boron tribromide in dichloromethane under nitrogen atmosphere. The reaction mixture is stirred for three hours between 0 °C and 15 °C. The reaction mixture is poured on a mixture of a saturated Sodium bicarbonate solution and ice. After dilution with 100 mL ethyl acetate the mixture is stirred vigorously for 20 minutes at room temperature. The organic phase is washed with water and brine and then dried (sodium sulfate). The solvent is evaporated and the residue is purified by chromatography (Lichroprep NH₂) eluting with dichloromethane/methanol. The chloro and the deschloro compounds can be separated at this stage. The title compound is isolated with a yield of 53.3 % (20.8 mg). The deschloro compound is isolated with a yield of 4.1 % (1.5 mg).

Title compound; ¹H-NMR (400 MHz, CD₃OD): δ = 1.55 (3H), 1.68 (3H), 2.01-2.18 (2H), 2.45 (3H), 5.15 (1H), 5.92 (1H), 6.55-6.65 (2H), 6.82 (1H), 7.06-7.22 (4H), 7.39-7.46 (2H), 8.25 (1H).

### Example 10

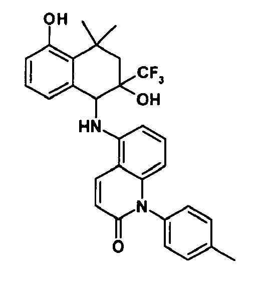

### 5-{[2,5-Dihydroxy-4,4-dimethyl-2-(trifluoromethyl)-1,2,3,4-tetrahydronaphthalene-1-yl]amino}-1-(4-methylphenyl)-1H-quinoline-2-one

The compound is isolated as further product of Example 9. The synthesis is described in Example 9.

Title compound; ¹H-NMR (300 MHz, CD₃OD): δ = 1.59 (3H), 1.70 (3H), 2.02-2.21 (2H), 2.51 (3H), 5.20 (1H), 5.97 (1H), 6.60-6.76 (3H), 6.85 (1H), 6.99 (1H), 7.12-7.29 (3H), 7.45-7.52 (2H), 8.32 (1H).

### Example 11

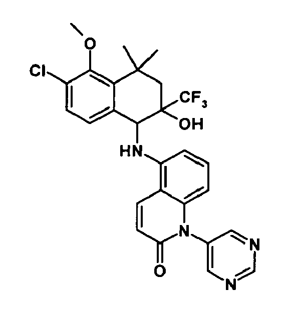

### 5-{[6-Chloro-2-hydroxy-5-methoxy-4,4-dimethyl-2-(trifluoromethyl)-1,2,3,4-tetrahydronaphthalene-1-yl]amino}-1-(1,3-pyrimidine-5-yl)-1H-quinoline-2-one

5-{[6-Chloro-2-hydroxy-5-methoxy-4,4-dimethyl-2-(trifluoromethyl)-1,2,3,4-tetrahydronaphthalene-1-yl]amino}-1H-quinoline-2-one (100 mg, 0.21 mmol), described in example 3, 1,3-pyrimidine-5-yl boronic acid (66.4 mg, 0.54 mmol), tetramethylethylendiamine (TMEDA) (0.096 mL, 0.64 mmol), copper(II)acetate (53.5 mg, 0.27 mmol) and 9.2 mL dichloromethane are stirred for 10 days at room temperature. The dark green mixture (no complete dissolution) is diluted with 100 mL ethyl acetate. The organic phase is washed with water and brine. After drying over sodium sulfate, the solvent is evaporated and the residue purified by chromatography (LiChroprep NH₂) eluting with dichloromethane/ methanol giving 17.3 mg (14.8%) of the title compound.

¹H-NMR (300 MHz, CD₃OD): δ = 1.51 (3H), 1.66 (3H), 2.11 (2H), 3.92 (3H), 5.22 (1H), 5.91 (1H), 6.60 (1H), 6.71 (1H), 7.05 (1H), 7.20 (1H), 7.29 (1H), 8.32 (1H), 8.80-8.90 (2H), 9.32 (1H).

### Example 12

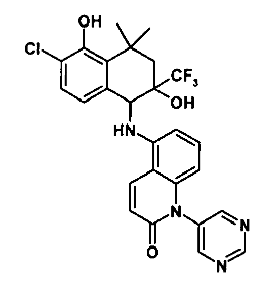

### 5-{[6-Chloro-2,5-dihydroxy-4,4-dimethyl-2-(trifluoromethyl)-1,2,3,4-tetrahydronaphthalene-1-yl]amino}-1-(1,3-pyrimidine-5-yl)-1H-quinoline-2-one

5-{[6-Chloro-2-hydroxy-5-methoxy-4,4-dimethyl-2-(trifluoromethyl)-1,2,3,4-tetrahydronaphthalene-1-yl]amino}-1-(1,3-pyrimidine-5-yl)-1H-quinoline-2-one

(42.2 mg, 0.077 mmol) is dissolved in 0.7 mL dichloromethane. At 0 °C 0.77 mL of an 1 M solution of boron tribromide in dichloromethane is added dropwise under nitrogen atmosphere. The reaction mixture is stirred for three hours between 0 °C and 15 °C and then poured on a mixture of a saturated sodium bicarbonate solution and ice. After dilution with 100 mL ethyl acetate the mixture is stirred vigorously for 20 minutes at room temperature. The organic phase is washed with water and brine and dried (sodium sulfate). After evaporation of the solvent the residue is purified by chromatography (Lichroprep) eluting with dichloromethane/ methanol. The title compound is isolated with a yield of 39.2 % (16.1 mg).

¹H-NMR (400 MHz, CD₃OD): δ = 1.55 (3H), 1.69 (3H), 2.02-2.19 (2H), 5.19 (1H), 5.90 (1H), 6.61 (1H), 6.71 (1), 6.83 (1H), 7.10 (1H), 7.29 (1H), 8.32 (1H), 8.80-8.90 (2H), 9.32 (1H).

### Example 13

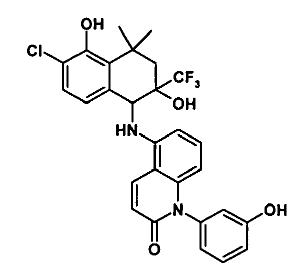

### 5-{[6-Chloro-2,5-dihydroxy-4,4-dimethyl-2-(trifluoromethyl)-1,2,3,4-tetrahydronaphthalene-1-yl]amino}-1-(3-hydroxyphenyl)-1H-quinoline-2-one

*5-{[6-Chloro-2-hydroxy-5-methoxy-4,4-dimethyl-2-(trifluoromethyl)-1,2,3,4-tetrahydronaphthalene-1-yl]amino}-1-(3-methoxyphenyl)-1H-quinoline-2-one*

5-{[6-Chloro-2-hydroxy-5-methoxy-4,4-dimethyl-2-(trifluoromethyl)-1,2,3,4-tetrahydronaphthalene-1-yl]amino}-1H-quinoline-2-one (167 mg, 0.36 mmol), described in example 3, 3-methoxyphenyl boronic acid (104.2 mg, 0.69 mmol), pyridine (0.55 mL, 6.85 mmol), copper(II)acetate (68.4 mg, 0.34 mmol) and 14.6 mL dichloromethane are stirred for three days at room temperature. In the beginning the solution is turquoise coloured and clear; later it turns into a dark green solution and a slight precipitate forms. This mixture is diluted with 150 mL ethyl acetate. The organic phase is washed with water and brine. After drying over sodium sulfate, the solvent is evaporated and the residue purified by chromatography (LiChroprep NH₂) eluting with dichloromethane/ methanol giving 26.3 mg of the title compound (contaminated).

*5-{[6-Chloro-2,5-dihydroxy-4,4-dimethy*/*-2-(trifluoromethyl)-1,2,3,4-tetrahydronaphthalene-1-yl]amino}-1-(3-hydroxyphenyl)-1H-quinoline-2-one*

5-{[6-Chloro-2-hydroxy-5-methoxy-4,4-dimethyl-2-(trifluoromethyl)-1,2,3,4-tetrahydronaphthalene-1-yl]amino}-1-(3-methoxyphenyl)-1H-quinoline-2-one (51 mg, 0.089 mmol) is dissolved in 0.9 mL dichloromethane. At 0 °C 0.89 mL of an 1 M solution of boron tribromide in dichloromethane is added dropwise under a nitrogen atmosphere. The reaction mixture is stirred for three hours between 0 °C and room temperature and then poured on a mixture of a saturated Sodium bicarbonate solution and ice. After dilution with 100 mL ethyl acetate the mixture is stirred vigorously for 20 minutes at room temperature. The organic phase is washed with water and brine and dried (sodium sulfate). The solvent is evaporated and the residue is purified by chromatography eluting with dichloromethane/ methanol. The title compound is isolated with a yield of 46.4 % (22.5 mg).

¹H-NMR (400 MHz, CD₃OD): δ = 1.57 (3H), 1.69 (3H), 2.02-2.18 (2H), 5.15 (1H), 6.00 (1H), 6.55-6.74 (4H), 6.83 (1H), 6.95 (1H), 7.11 (1H), 7.21 (1H), 7.42 (1H); 8.26 (1H).

### Example 14

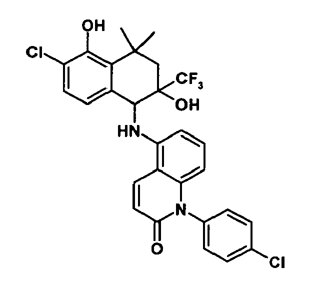

### 5-{[6-Chloro-2,5-dihydroxy-4,4-dimethyl-2-(trifluoromethyl)-1,2,3,4-tetrahydronaphthalene-1-yl]amino}-1-(4-chlorophenyl)-1H-quinoline-2-one

*5-{[6-Chloro-2-hydroxy-5-methoxy-4,4-dimethyl-2-(trifluoromethyl)-1,2,3,4-tetrahydronaphthalene-1-yl]amino}-1-(4-chlorophenyl)-1H-quinoline-2-one*

A mixture of 5-{[6-Chloro-2-hydroxy-5-methoxy-4,4-dimethyl-2-(trifluoromethyl)-1,2,3,4-tetrahydronaphthalene-1-yl]amino}-1H-quinoline-2-one and the corresponding deschloro compound (160 mg, 0.34 mmol), described in example 3, 4-chlorophenyl boronic acid (107.2 mg, 0.69 mmol), pyridine (0.55 mL, 6.85 mmol), copper(II)acetate (68.4 mg, 0.34 mmol) and 14.6 mL dichloromethane are stirred for three days at room temperature. The turquoise coloured solution is diluted with 150 mL ethyl acetate. The organic phase is washed with water and brine. After drying over sodium sulfate, the solvent is evaporated and the residue purified by chromatography (LiChroprep NH₂) eluting with dichloromethane/methanol giving 52.5 mg (26.5%) of the title compound as a mixture.

*5-{[6-Chloro-2,5-dihydroxy-4,4-dimethyl-2-(trifluoromethyl)-1,2,3,4-tetrahydronaphthalene-1-yl]amino}-1-(4-chlorophenyl)-1H-quinoline-2-one*

The mixture of 5-{[6-Chloro-2-hydroxy-5-methoxy-4,4-dimethyl-2-(trifluoromethyl)-1,2,3,4-tetrahydronaphthalene-1-yl]amino}-1-(4-chlorophenyl)-1H-quinoline-2-one and its deschloro derivative (51 mg, 0.088 mmol) is dissolved in 0.8 mL dichloromethane. At 0 °C 0.88 mL of an 1 M solution of boron tribromide in dichloromethane is added dropwise under nitrogen atmosphere. The reaction mixture is stirred for three hours between 0 °C and room temperature and then poured on a mixture of a saturated sodium bicarbonate solution and ice. After dilution with 100 mL ethyl acetate the mixture is stirred vigorously for 20 minutes at room temperature. The organic phase is washed with water and brine and dried (sodium sulfate ). The solvent is evaporated and the residue is purified by chromatography (LiChroprep) eluting with dichloromethane/ methanol. The chloro compound is isolated with a yield of 89.4 % (44.5 mg). The corresponding deschloro derivate is obtained with a yield of 9.4 % (4.4 mg).

Title compound; ¹H-NMR (300 MHz, CD₃OD): δ = 1.56 (3H), 1.69 (3H), 2.00-2.18 (2H), 5.15 (1H), 5.90 (1H), 6.59 (1H), 6.63 (1H), 6.81 (1H), 7.09 (1H), 7.15-7.32 (3H), 7.58-7.65 (2H), 8.28 (1H).

### Example 15

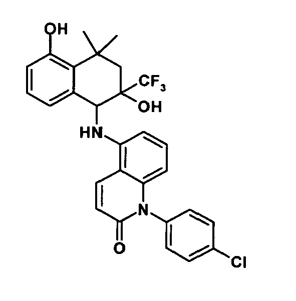

### 5-{[2,5-Dihydroxy-4,4-dimethyl-2-(trifluoromethyl)-1,2,3,4-tetrahydronaphthalene-1-yl]amino}-1-(4-chlorophenyl)-1H-quinoline-2-one

The compound is isolated as further product of example 14. The synthesis is described in example 14.

Title compound; ¹H-NMR (300 MHz, CD₃OD): δ = 1.58 (3H), 1.71 (3H), 2.01-2.21 (2H), 5.19 (1H), 5.94 (1H), 6.59-6.75 (3H), 6.82 (1H), 6.98 (1H), 7.21-7.39 (3H), 7.62-7.71 (2H), 8.32 (1H).

### Example 16

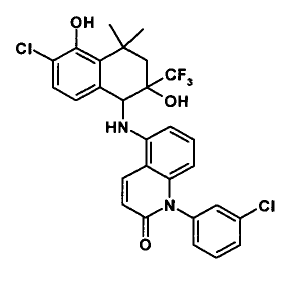

### 5-{[6-Chloro-2,5-dihydroxy-4,4-dimethyl-2-(trifluoromethyl)-1,2,3,4-tetrahydronaphthalene-1-yl]amino}-1-(3-chlorophenyl)-1H-quinoline-2-one

*5-{[6-Ch*/*oro-2-hydroxy-5-methoxy-4,4-dimethyl-2-(trifluoromethyl)-1,2,3,4-tetrahydronaphthalene-1-yl]amino}-1-(3-chlorophenyl)-1H-quinoline-2-one*

A mixture of 5-{[6-Chloro-2-hydroxy-5-methoxy-4,4-dimethyl-2-(trifluoromethyl)-1,2,3,4-tetrahydronaphthalene-1-yl]amino}-1H-quinoline-2-one and its deschloro derivative (160 mg, 0.34 mmol), described in example 3, 3-chlorophenyl boronic acid (107.2 mg, 0.69 mmol), pyridine (0.55 mL, 6.85 mmol), copper(II)acetate (68.4 mg, 0.34 mmol) and 14.6 mL dichloromethane is stirred for three days at room temperature. The turquoise coloured solution is diluted with 150 mL ethyl acetate. The organic phase is washed with water and brine. After drying over sodium sulfate, the solvent is evaporated and the residue purified by chromatography (Isolute NH₂) eluting with dichloromethane/ methanol giving 31.5 mg (15.9%) of the title compound as a mixture.

*5-{[6-Chloro-2,5-dihydroxy-4,4-dimethyl-2-(trifluoromethyl)-1,2,3,4-tetrahydronaphthalene-1-yl]amino}-1-(3-chlorophenyl)-1H-quinoline-2-one*

The mixture of 5-{[6-Chloro-2-hydroxy-5-methoxy-4,4-dimethyl-2-(trifluoromethyl)-1,2,3,4-tetrahydronaphthalene-1-yl]amino}-(3-chlorophenyl)-1H-quinoline-2-one and its deschloro derivative (30 mg, 0.052 mmol) is dissolved in 0.5 mL dichloromethane. At 0 °C 0.52 mL of an 1 M solution of boron tribromide in dichloromethane is added dropwise under nitrogen atmosphere. The reaction mixture is stirred for three hours between 0 °C and room temperature and then poured on a mixture of a saturated sodium bicarbonate solution and ice. After dilution with 100 mL ethyl acetate the mixture is stirred vigorously for 20 minutes at room temperature. The organic phase is washed with water and brine and then dried (sodium sulfate). The solvent is evaporated and the residue is purified by chromatography (Isolute NH₂) eluting with dichloromethane/ methanol. The chloro compound is obtained with a yield of 73.8 % (21.6 mg), the corresponding deschloro derivate with a yield of 6.6 % (1.8 mg).

Title compound; ¹H-NMR (300 MHz, CD₃OD): δ = 1.55 (3H), 1.69 (3H), 2.01-2.18 (2H), 5.15 (1H), 5.90 (1H), 6.59 (1H), 6.65 (1H), 6.81 (1H), 7.09 (1H), 7.18-7.29 (2H), 7.36 (1H), 7.52-7.65 (2H), 8.28 (1H).

### Example 17

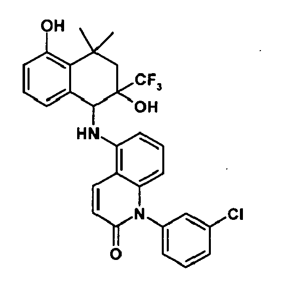

### 5-{[2,5-Dihydroxy-4,4-dimethyl-2-(trifluoromethyl)-1,2,3,4-tetrahydronaphthalene-1-yl]amino}-1-(3-chlorophenyl)-1H-quinoline-2-one

The compound is isolated as further product of Example 16. The synthesis is described in Example 16.

Title compound; ¹H-NMR (300 MHz, CD₃OD): δ = 1.59 (3H), 1.72 (3H), 2.01-2.21 (2H), 5.20 (1H), 5.94 (1H), 6.59-6.75 (3H), 6.82 (1H), 6.98 (1H), 7.22-7.35 (2H), 7.42 (1H), 7.59-7.71 (2H), 8.32 (1H).

### Example 18

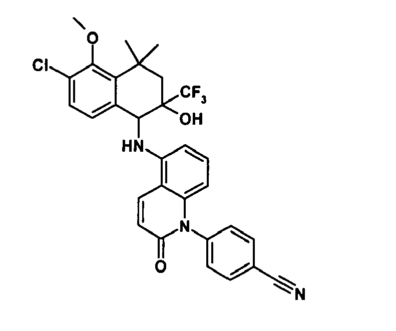

### 5-{[6-Chloro-2-hydroxy-5-methoxy-4,4-dimethyl-2-(trifluoromethyl)-1,2,3,4-tetrahydronaphthalene-1-yl]amino}-1-(4-cyanophenyl)-1H-quinoline-2-one

5-{[6-Chloro-2-hydroxy-5-methoxy-4,4-dimethyl-2-(trifluoromethyl)-1,2,3,4-tetrahydronaphthalene-1-yl]amino}-1H-quinoline-2-one (400 mg, 0.86 mmol), described in example 3, 4-cyanophenyl boronic acid (251.8 mg, 1.71 mmol), pyridine (1.38 mL, 17.13 mmol), copper(II)acetate (171 mg, 0.86 mmol) and 36.5 mL dichloromethane are stirred for three days at room temperature. The dark green solution is diluted with 50 mL ethyl acetate. The organic phase is washed with water and brine. After drying over sodium sulfate, the solvent is evaporated and the residue is purified by chromatography (Isolute NH₂) eluting with dichloromethane/ methanol. 94.9 mg (19.1%) of the title compound are isolated.

¹H-NMR (300 MHz, CD₃OD): δ = 1.50 (3H), 1.65 (3H), 2.11 (2H), 3.91 (3H), 5.19 (1H), 5.85 (1H), 6.59 (1H), 6.68 (1H), 7.05 (1H), 7.13-7.29 (2H), 7.45-7.59 (2H), 7.95-8.04 (2H), 8.29 (1H).

### Example 19

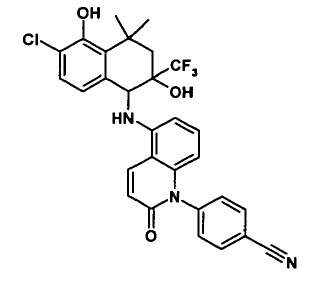

### 5-{[6-Chloro-2,5-dihydroxy-4,4-dimethyl-2-(trifluoromethyl)-1,2,3,4-tetrahydronaphthalene-1-yl]amino}-1-(4-cyanophenyl)-1H-quinoline-2-one

5-{[6-Chloro-2-hydroxy-5-methoxy-4,4-dimethyl-2-(trifluoromethyl)-1,2,3,4-tetrahydronaphthalene-1-yl]amino}-1-(4-cyanophenyl)-1H-quinoline-2-one (30 mg, 0.052 mmol) is dissolved in 0.5 mL dichloromethane. At 0 °C 0.52 mL of an 1 M solution of boron tribromide in dichloromethane is added dropwise under nitrogen atmosphere. The reaction mixture is stirred for four hours between 0 °C and room temperature and then poured on a mixture of a saturated Sodium bicarbonate solution and ice. After dilution with 100 mL ethyl acetate the mixture is stirred vigorously for 20 minutes at room temperature. The organic phase is washed with water and brine and dried (sodium sulfate). The solvent is evaporated and the residue is purified by chromatography (Isolute NH₂) eluting with dichloromethane/ methanol. The title compound is obtained with a yield of 79.1 % (71.7 mg).

¹H-NMR (400 MHz, CD₃OD): δ = 1.55 (3H), 1.68 (3H), 2.01-2.18 (2H), 5.15 (1H), 5.85 (1H), 6.59 (1H), 6.68 (1H), 6.82 (1H), 7.10 (1H), 7.22 (1H), 7.45-7.57 (2H), 7.95-8.02 (2H), 8.29 (1H).

### Example 20

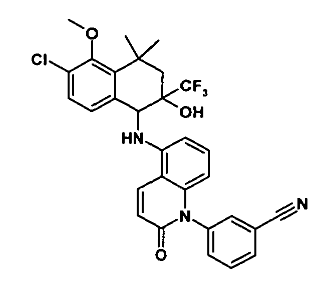

### 5-{[6-Chloro-2-hydroxy-5-methoxy-4,4-dimethyl-2-(trifluoromethyl)-1,2,3,4-tetrahydronaphthalene-1-yl]amino}-1-(3-cyanophenyl)-1H-quinoline-2-one

5-{[6-Chloro-2-hydroxy-5-methoxy-4, 4-dimethyl-2-(trifluoromethyl)-1,2,3,4-tetrahydronaphthalene-1-yl]amino}-1H-quinoline-2-one (400 mg, 0.86 mmol), described in example 3, 3-cyanophenyl boronic acid (251.8 mg, 1.71 mmol), pyridine (1.38 mL, 17.13 mmol), copper(II)acetate (171 mg, 0.86 mmol) and 36.5 mL dichloromethane are stirred for three days at room temperature. The dark green solution is diluted with 150 mL ethyl acetate. The organic phase is washed with water (twice, each 20 mL) and brine (20 mL). After drying over sodium sulfate, the solvent is evaporated and the residue is purified by chromatography (Isolute NH₂) eluting with dichloromethane/ methanol. The title compound is isolated with a yield of 15.9% (77.4 mg).

¹H-NMR (400 MHz, CD₃OD): δ = 1.51 (3H), 1.66 (3H), 2.10 (2H), 3.93 (3H), 5.19 (1 H), 5.85 (1H), 6.61 (1H), 6.68 (1H), 7.05 (1H), 7.17-7.29 (2H), 7.63 (1H), 7.70-7.82 (2H), 7.92 (1H), 8.29 (1H).

### Example 21

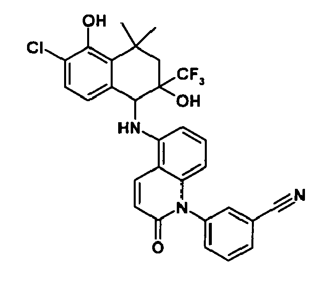

### 5-{[6-Chloro-2,5-dihydroxy-4,4-dimethyl-2-(trifluoromethyl)-1,2,3,4-tetrahydronaphthalene-1-yl]amino}-1-(3-cyanophenyl)-1H-quinoline-2-one

5-{[6-Chloro-2-hydroxy-5-methoxy-4, 4-dimethyl-2-(trifluoromethyl)-1,2,3,4-tetrahydronaphthalene-1-yl]amino}-1-(3-cyanophenyl)-1H-quinoline-2-one (75 mg, 0.13 mmol) is dissolved in 1.3 mL dichloromethane. At 0 °C 1.32 mL of an 1 M solution of boron tribromide in dichloromethane is added dropwise under nitrogen atmosphere. The reaction mixture is stirred for four hours between 0 °C and room temperature and then poured on a mixture of a saturated Sodium bicarbonate solution and ice. After dilution with 50 mL ethyl acetate the mixture is stirred vigorously for 20 minutes at room temperature. The organic phase is washed with water and brine and dried (sodium sulfate). The solvent is evaporated and the residue is purified by chromatography (Isolute NH₂) eluting with dichloromethane/ methanol. The title compound is obtained with a yield of 75.3 % (55.1 mg).

¹H-NMR (300 MHz, CD₃OD): δ = 1.55 (3H), 1.69 (3H), 2.01-2.18 (2H), 5.17 (1H), 5.85 (1H), 6.60 (1H), 6.68 (1H), 6.82 (1H), 7.10 (1H), 7.23 (1H), 7.63 (1H), 7.70-7.85 (2H), 7.92 (1H), 8.29 (1H).

### Example 22

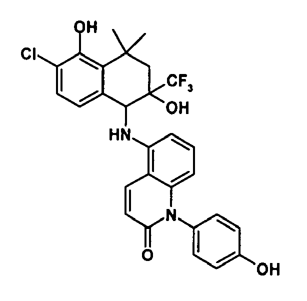

### 5-{[6-Chloro-2,5-dihydroxy-4,4-dimethyl-2-(trifluoromethyl)-1,2,3,4-tetrahydronaphthalene-1-yl]amino}-1-(4-hydroxyhenyl)-1H-quinoline-2-one

*5-{[6-Chloro-2-hydroxy-5-methoxy-4, 4-dimethyl-2-(trifluoromethyl)-1,2,3,4-tetrahydronaphthalene-1-yl]amino}-1-(4-methoxyphenyl)-1H-quinoline-2-one*

5-{[6-Chloro-2-hydroxy-5-methoxy-4, 4-dimethyl-2-(trifluoromethyl)-1,2,3,4-tetrahydronaphthalene-1-yl]amino}-1H-quinoline-2-one (300 mg, 0.64 mmol), described in example 3, 4-methoxyphenyl boronic acid (195.3 mg, 1.29 mmol), pyridine (1.04 mL, 12.85 mmol), copper(II)acetate (128.3 mg, 0.64 mmol) and 27.4 mL dichloromethane are stirred for three days at room temperature. The colour of the reaction mixture has turned from dark green into dark brown. The solution is diluted with 150 mL ethyl acetate and the organic phase is washed with water (twice, each 20 mL) and brine (20 mL). After drying over sodium sulfate, the solvent is evaporated and the residue is purified by chromatography (Isolute NH₂) eluting with dichloromethane/ methanol. The arylated product is isolated with a yield of 5.3% (19.6 mg).

*5-{[6-Chloro-2,5-dihydroxy-4, 4-dimethyl-2-(trifluoromethyl)-1,2,3,4-tetrahydronaphthalene-1-yl]amino}-1-(4-hydroxyhenyl)-1H-quinoline-2-one*

The aforementioned product 5-{[6-Chloro-2-hydroxy-5-methoxy-4, 4-dimethyl-2-(trifluoromethyl)-1,2,3,4-tetrahydronaphthalene-1-yl]amino}-1-(4-methoxyphenyl)-1H-quinoline-2-one (18.3 mg, 0.032 mmol) is dissolved in 0.3 mL dichloromethane. At 0 °C 0.32 mL of an 1M solution of boron tribromide in dichloromethane is added dropwise under nitrogen atmosphere. The reaction mixture is stirred for four hours between 0 °C and room temperature and then poured on a mixture of a saturated sodium bicarbonate solution and ice. After dilution with 50 mL ethyl acetate the mixture is stirred vigorously for 20 minutes at room temperature. The organic phase is washed with water and brine and dried (sodium sulfate ). After evaporation of the solvent the residue is purified by chromatography (Isolute NH₂) eluting with dichloromethane/ methanol giving the title compound (10.8 mg, 62.1%).

¹H-NMR (300 MHz, CD₃OD): δ = 1.55 (3H), 1.69 (3H), 2.01-2.18 (2H), 5.12 (1H), 6.00 (1H), 6.55-6.65 (2H), 6.82 (1H), 6.95-7.12 (5H), 7.20 (1 H), 8.23 (1 H).

### Example 23

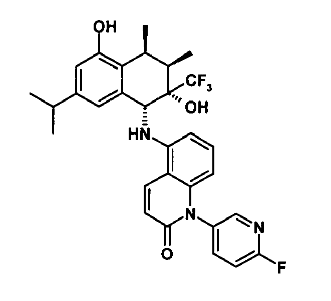

### (1RS, 2SR, 3RS, 4SR)-5-{[2,5-Dihydroxy-7-isopropyl-3,4-dimethyl-2-(trifluoromethyl)-1,2,3,4-tetrahydronaphthalene-1-yl]amino}-1-(6-fluoropyridin-3yl)-1H-quinoline-2-one

### (3-Isopropylphenyl) acetate

3-isopropylphenol (40 g, 293.7 mmol) is dissolved in 287 mL dichloromethane and 32.2 mL (399.4 mmol) pyridine is added at room temperature. Thereby the temperature rises to 29 °C. The reaction mixture is stirred for 15 minutes. After cooling of the mixture to 10 - 15 °C acetyl chloride (26.2 mL , 367.1 mmol) is added within 20 minutes. After stirring overnight at room temperature the reaction mixture is poured on a mixture of 190 mL 2N HCl and ice. After 20 minutes of stirring the dichloromethane phase is separated and the aqueous phase extracted with 500 mL dichloromethane. The combined organic extracts are washed with brine and dried over sodium sulfate. The solvent is evaporated and the residue purified by chromatography (eluents ethyl acetate/ hexane) yielding 48.45 g (92.6%) of the desired ester.

¹H-NMR (400 MHz, CDCl₃): δ = 1.28 (6H), 2.30 (3H), 2.92 (1H), 6.88-6.97 (2H), 7.10 (1H), 7.29 (1H).

### 4'-Isopropy/-2'-hydroxy acetophenone

To aluminium trichloride (35.2 g, 263.7 mmol) in 110 mL 1.2-dichlorobenzene is added dropwise within 15 minutes (3-isopropylphenyl) acetate (48.5 g, 271.8 mmol) in 94 mL 1.2-dichlorobenzene. The temperature rises to 40 °C. After stirring at 100 °C for 17 hours the reaction mixture is allowed to cool down and poured on a mixture of 230 mL 4N HCl and ice. After extraction with diethylether (trice with 300 mL each) the combined organic extracts are washed with brine and dried over sodium sulfate. The solvent is removed and the residue purified by chromatography eluting with ethyl acetate/ hexane. The title compound is obtained with a yield of 84.6% (41g).

¹H-NMR (300 MHz, CDCl₃): δ = 1.25 (6H), 2.60 (3H), 2.89 (1H), 6.79 (1H), 6.85 (1H), 7.65 (1H), 12.29 (1H).

### 4'-Isopropyl-2'-methoxy acetophenone

4'-Isopropyl-2'-hydroxy acetophenone (33.4 g, 187.4 mmol) is dissolved in acetone (233 mL). K₂CO₃ (51.8 g, 374.8 mmol) and methyliodide (23.3 mL, 374.8 mmol) are added at room temperature. After boiling for three days the reaction mixture is filtered via a glass microfibre filter and the residue washed with cold acetone. The solvent is removed and the residue purified by chromatography eluting with ethyl acetate/ hexane. The title compound is obtained with a yield of 88.4% (31.85 g).

¹H-NMR (400 MHz, CDCl₃): δ = 1.28 (6H), 2.60 (3H), 2.93 (1H), 3.92 (3H), 6.81 (1H), 6.88 (1H), 7.72 (1H).

### 4-Isopropyl-2-methoxy-1-(1-methyl-propenyl)benzene

Ethyl triphenyl phosphonium bromide (67.5 g, 181.8 mmol) are given in 675 mL tetrahydrofurane. Potassium hexamethyldisilazide (363.6 mL of a 0.5 M solution in toluene, 181.8 mmol) is added within 30 minutes between -5 °C and 0 °C to the white suspension. The red suspension is stirred for two and a half hour at room temperature. 4'-Isopropyl-2'-methoxy acetophenone (23.3 g, 121.2 mmol), dissolved in 280 mL tetrahydrofurane, is added within 35 minutes to the ylid. The temperature rises to 29 °C. The orange-brown reaction mixture is stirred for four days at room temperature and then poured on 100 mL water. After addition of ethyl acetate (250 mL) the reaction mixture is stirred for 10 minutes. The organic phase is separated and washed with water (40 mL) and brine (40 mL). The mixture is dried (sodium sulfate) and the solvent removed. The residue is purified by chromatography (eluents ethyl acetate/ hexane) yielding more than 100% of the product (26.44 g) as an E/Z mixture.

¹H-NMR (300 MHz, CDCl₃): δ = 1.20-1.35 (6H), 1.42-1.52 (3H), 1.95-2.02 (3H), 2.91 (1H), 3.84 (3H), 5.49-5.65 (1 H), 6.73-6.85 (2H), 6.97 (1 H).

### Ethyl-4-(4-isopropyl-2-methoxyphenyl)-2-hydroxy-3-methyl-2-(trifluoromethyl)-pent-4-enoate

Ytterbium(III)-trifluoromethansulfonate (3.25 g, 5.23 mmol) is given in 116 mL dichloromethane. Ethyl trifluoropyruvate (9.55 mL, 78.5 mmol) is added dropwise at room temperature. After addition of 4-isopropyl-2-methoxy-1-(1-methyl-propenyl)benzene (10.7 g, 52.4 mmol), dissolved in 29.5 mL dichloromethane, the reaction mixture is stirred for two days at room temperature. A second reaction is carried out In parallel with the same amounts of reagents and under the same conditions. Water (100mL) is added to each reaction mixture. For the further work up both reactions have been combined. The dichloromethane phase is separated and the aqueous phase extracted once more with dichloromethane (300 mL). The combined organic phases are washed with water (100 mL) and brine (100 mL) and dried (sodium sulfate ). After evaporation of the solvent the residue is purified several times by chromatography eluting with ethyl acetate/ hexane yielding in total 16.9 g (43.2%) of the desired compound which contains ca. 20% of the corresponding ethyl-4-(4-isopropyl-2-methoxyphenyl)-2-hydroxy-2-(trifluoromethyl)-hex-4-enoate.

### 4-(4-Isopropyl-2-methoxyphenyl)-2-hydroxy-3-methyl-2-(trifluoromethyl)-pent-4-en-1-ol

The aforementioned ethyl-4-(4-isopropyl-2-methoxyphenyl)-2-hydroxy-3-methyl-2-(trifluoromethyl)-pent-4-enoate (10.2 g, 27.2 mmol) is dissolved in diethylether (264 mL). Lithiumaluminiumhydride (2.07 g, 54.5 mmol) is added in portions within 30 minutes at 5 °C. The reaction mixture is stirred for two and a half hours. Saturated Sodium bicarbonate solution (12 mL) is added dropwise to the reaction mixture. The ice bath is removed and the mixture stirred vigorously for two hours at room temperature. After sucking off the precipitation via a glass microfibre filter the residue is washed with diethylether (200 mL). The organic phase is washed twice with brine (50 mL each) and dried (sodium sulfate). After chromatography eluting with ethyl acetate/ hexane the title compound is obtained jointly with 4-(4-isopropyl-2-methoxyphenyl)-2-hydroxy-2-(trifluoromethyl)-hex-4-en-1-ol in a ratio of 84:16% (overall yield 55.2%, 5.05 g).

### 4-(4-Isopropyl-2-methoxyphenyl)-2-hydroxy-3-methyl-2-(trifluoromethyl)-pentan-1-ol

4-(4-Isopropyl-2-methoxyphenyl)-2-hydroxy-3-methyl-2-(trifluoromethyl)-pent-4-en-1-ol (5 g, 15.04 mmol) is dissolved in ethanol (100 mL). After addition of Pd/ 10% on charcoal (1.5 g), the reaction mixture is charged with hydrogen (for four hours via a balloon). The reaction mixture is sucked off via a glass microfibre filter and the precipitate washed with ethanol. After removal of the solvent, the crude residue (as a mixture) is used in the next step.

### 4-(4-Isopropyl-2-methoxyphenyl)-2-hydroxy-3-methyl-2-(trifluoromethyl)-pentanal

4-(4-Isopropyl-2-methoxyphenyl)-2-hydroxy-3-methyl-2-(trifluoromethyl)-pentan-1-ol (2 g, 6 mmol) is dissolved in dichloromethane (61 mL). After addition of dimethylsulfoxide (20.5 mL) and triethylamine (4.15 mL), SO₃/ pyridine complex (2.38 g, 14.95 mmoL) is added in portions within 25 minutes. After stirring for three and a half hours the reaction mixture is poured on a mixture of saturated ammonium chloride solution and ice. The mixture is extracted twice with diethylether (150 mL each) and the combined organic extracts are washed with brine and dried (sodium sulfate). After evaporation of the solvent the residue is purified by chromatography eluting with diethylether/ hexane. The desired compound is obtained with a yield of 86% (1.71 g) jointly with the corresponding 4-(4-isopropyl-2-methoxyphenyl)-2-hydroxy-2-(trifluoromethyl)-hexanal.

### 5-{[4-(4-Isopropyl-2-methoxyphenyl)-2-hydroxy-3-methyl-2-(trifluoromethyl)pentyliden]amino}1H-quinoline-2-one

The above described mixture, 4-(4-isopropyl-2-methoxyphenyl)-2-hydroxy-3-methyl-2-(trifluoromethyl)-pentanal and 4-(4-isopropyl-2-methoxyphenyl)-2-hydroxy-2-(trifluoromethyl)-hexanal (600 mg, 1.8 mmol), 5-amino-1H-quinoline-2-one (287.4 mg, 1.79 mmol) and 2.67 mL acetic acid are stirred together for three days. Toluene is added and the reaction mixture is evaporated. This procedure is repeated twice. The residue is purified by chromatography eluting with ethyl acetate/ hexane. The desired imine is obtained as a mixture with a yield of 86.1% (732.8 mg).

### 5-{[2,5-Dihydroxy-7-isopropyl-3,4-dimethyl-2-(trifluoromethyl)-1,2,3,4-tetrahydronaphthalene-1-yl]amino}-1H-quinoline-2-one

5-{[4-(4-Isopropyl-2-methoxyphenyl)-2-hydroxy-3-methyl-2-trifluoromethyl) pentyliden] amino} 1H-quinoline-2-one (580 mg, 1.22 mmol) is dissolved in 5.6 mL dichloromethane. boron tribromide (12.22 mL of an 1M solution in dichloromethane) is added dropwise at -10 °C under a nitrogen atmosphere. After stirring for three and a half hours between -10 and +5 °C the reaction mixture is poured on saturated Sodium bicarbonate solution and ice. The mixture is diluted with 100 mL ethyl acetate and vigorously stirred for 20 minutes. The ethyl acetate phase is separated and the aqueous phase washed once with 50 mL ethyl acetate. The combined organic extracts are washed with water and brine. After drying over sodium sulphate the solvent is evaporated. The residue is purified by column chromatography eluting with dichloromethane/ methanol to give the title compound (284.1 mg, 50.4%).

¹H-NMR (300 MHz, CD₃OD): δ = 1.01 (6H), 1.13 (3H), 1.40 (3H), 2.39 (1H), 2.59 (1H), 3.44 (1H), 5.06 (1H), 6.47 (1H), 6.52-6.60 (2H), 6.61-6.72 (2H), 7.38 (1H), 8.13 (1H).

### (1RS, 2SR, 3RS, 4SR)-5-{[2,5-Dihydroxy-7-isopropyl-3,4-dimethyl-2-(trifluoromethyl)-1,2,3,4-tetrahydronaphthalene-1-yl]amino}-1-(6-fluoropyridine-3yl)-1H-quinoline-2-one

5-{[2,5-Dihydroxy-7-isopropyl-3,4-dimethyl-2-(trifluoromethyl)-1,2,3,4-tetrahydronaphthalene-1-yl]amino}-1H-quinoline-2-one (40 mg, 0.087 mmol), 6-fluoropyridine-3-yl-boronic acid (12.2 mg, 0.087 mmol), pyridine (0.14 mL, 1.74 mmol), copper(II)acetate (17.3 mg, 0.087 mmol) and 3.7 mL dichloromethane are stirred for five days at room temperature. The turquoise coloured solution is diluted with 50 mL ethyl acetate. The organic phase is washed with water (10 mL) and brine (10 mL). After drying over sodium sulfate, the solvent is evaporated and the residue purified by chromatography (LiChroprep NH₂) eluting with dichloromethane/ methanol. The title compound is isolated with a yield of 28% (13.5 mg).

¹H-NMR (400 MHz, CD₃OD): δ = 1.06 (6H), 1.15 (3H), 1.40 (3H), 2.39 (1H), 2.61 (1H), 3.45 (1H), 5.10 (1H), 5.93 (1H), 6.53-6.64 (3H), 6.73 (1H), 7.26-7.38 (2H), 7.96 (1H), 8.20 (1H), 8.26 (1H).

### Example 24

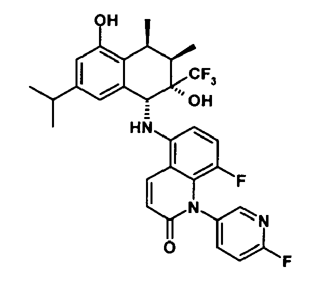

### (1RS, 2SR, 3RS, 4SR)-5-{[2,5-Dihydroxy-7-isopropyl-3,4-dimethyl-2-(trifluoromethyl)-1,2,3,4-tetrahydronaphthalene-l-yl]amino}-8-fluoro-1-(6-fluoropyridin-3yl)-1H-quinoline-2-one

### 5-{[4-(4-Isopropyl-2-methoxyphenyl)-2-hydroxy-3-methyl-2-trifluoromethyl)pentyliden]amino}-8-fluoro-1H-quinoline-2-one

The mixture of 4-(4-Isopropyl-2-methoxyphenyl)-2-hydroxy-3-methyl-2-(trifluoromethyl)-pentanal and 4-(4-isopropyl-2-methoxyphenyl)-2-hydroxy-2-(trifluoromethyl)-hexanal (600 mg, 1.8 mmol), described in example 23, 5-amino-8-fluoro-1H-quinoline-2-one (319.7 mg, 1.79 mmol) and 2.67 mL acetic acid are stirred together for three days. Toluene is added and the reaction mixture is evaporated. This procedure is repeated twice. The residue is purified by chromatography eluting with ethyl acetate/ hexane. The desired imine is obtained as a mixture with a yield of 77.6% (685.9 mg).

### (1RS, 2SR, 3RS, 4SR)-5-{[2,5-Dihydroxy-7-isopropyl-3,4-dimethyl-2-(trifluoromethyl)-1,2,3,4-tetrahydronaphthalene-1-yl]amino}-8-fluoro-1H-quinoline-2-one

The mixture of 5-{[4-(4-Isopropyl-2-methoxyphenyl)-2-hydroxy-3-methyl-2-trifluoromethyl) pentyliden]amino}-8-fluoro-1H-quinoline-2-one and 5-{[4-(4-Isopropy)-2-methoxyphenyl)-2-hydroxy-2-trifluoromethyl)hexyliden]amino}-8-fluoro-1H-quinoline-2-one (680 mg, 1.38 mmol) is dissolved in 6.8 mL dichloromethane. Boron tribromide (13.81 mL of an 1M solution in dichloromethane) is added dropwise at -10 °C under a nitrogen atmosphere. After stirring for three and a half hours between -10 and +5 °C the reaction mixture is poured on a mixture of saturated sodium bicarbonate solution and ice. The mixture is diluted with 100 mL ethyl acetate and vigorously stirred for 20 minutes. The organic phase is separated and the aqueous phase washed once with 50 mL ethyl acetate. The combined organic extracts are washed with water and brine. After drying with sodium sulfate the solvent is evaporated. The residue is purified by column chromatography eluting with dichloromethane/ methanol and subsequent HPLC to give the title compound (359.1 mg = 49.8%).

¹H-NMR (300 MHz, CD₃OD): δ = 1.05 (6H), 1.13 (3H), 1.39 (3H), 2.38 (1H), 2.61 (1H), 3.42 (1H), 4.99 (1H), 6.50 (1H), 6.51-6.62 (3H), 7.22 (1H), 8.11 (1H).

### (1RS, 2SR, 3RS, 4SR)-5-{[2,5-Dihydroxy-7-isopropyl-3,4-dimethyl-2-(trifluoromethyl)-1,2,3,4-tetrahydronaphthalene-1-yl]amino}-8-fluoro-1-(6-fluoropyridine-3yl)-1H-quinolin-2-one

5-{[2,5-Dihydroxy-7-isopropyl-3,4-dimethyl-2-(trifluoromethyl)-1,2,3,4-tetrahydronaphthalene-1-yl]amino}-8-fluoro-1H-quinoline-2-one (40 mg, 0.087 mmol), 6-fluoropyridine-3-yl-boronic acid (24.48 mg, 0.17 mmol), pyridine (0.14 mL, 1.74 mmol), copper(II)acetate (17.34 mg, 0.087 mmol) and 3.7 mL dichloromethane are stirred for 26 hours at room temperature. Because the starting material has not completely reacted the same amounts of boronic acid and copper(II)acetate are added and the reaction mixture is stirred for another two days. The turquoise coloured solution is diluted with 50 mL ethyl acetate. The organic phase is washed with water (10 mL) and brine (10 mL). After drying over sodium sulfate, the solvent is evaporated and the residue purified by chromatography (LiChroprep NH₂) eluting with dichloromethane/ methanol. The main product with 6.5 mg (13.6%) is (5RS, 6SR, 7RS, 8SR)-5-[8-fluoro-2-(6-fluoropyridine-3-yloxy)quinoline-5-ylamino]-3-isopropyl-7,8-dimethyl-6 - (trifluoromethyl)-5,6,7,8-tetrahydronaphthalene-1,6-diol.

The title compound is isolated with a yield of 6.88% (3.3 mg, contaminated).

¹H-NMR (400 MHz, CD₃OD): δ = 1.06 (6H), 1.13 (3H), 1.40 (3H), 2.39 (1H), 2.62 (1H), 3.43 (1H), 5.01 (1H), 6.55-6.62 (3H), 6.72 (1H), 7.10-7.25 (2H), 7.97 (1H), 8.17-8.29 (2H).

### Example 25

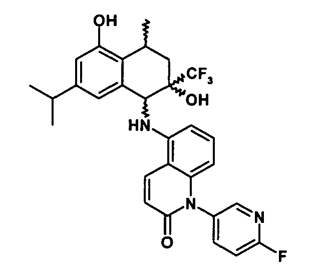

### 5-{[2,5-Dihydroxy-7-isopropyl-4-methyl-2-(trifluoromethyl)-1,2,3,4-tetrahydronaphthalene-1-yl]amino}-1-(6-fluoropyridin-3yl)-1H-quinoline-2-one

5-{[2,5-Dihydroxy-7-isopropyl-4-methyl-2-(trifluoromethyl)-1,2,3,4-tetrahydronaphthalene-1-yl] amino}-1H-quinoline-2-one (42 mg, 0.094 mmol), prepared analogously to the described example 23, 6-fluoropyridine-3-yl-boronic acid (26.5 mg, 0.19 mmol), pyridine (0.15 mL, 1.88 mmol), copper(II)acetate (18.8 mg, 0.094 mmol) and 4 mL dichloromethane are stirred for 26 hours at room temperature. The turquoise coloured solution is diluted with 50 mL ethyl acetate. The organic phase is washed with water (10 mL) and brine (10 mL). After drying over sodium sulfate, the solvent is evaporated and the residue purified by chromatography (LiChroprep NH₂) eluting with dichloromethane/ methanol followed by a preparative TLC (eluting with dichloromethane/ methanol). The title compound is obtained with a yield of 25.7% (13.1 mg) as a mixture of stereoisomers (ratio 85:15). The spectroscopic data of the major stereoisomer is described.

¹H-NMR (400 MHz, CD₃OD): δ = 1.08 (6H), 1.53 (3H), 2.15 (1H), 2.25 (1H), 2.62 (1H), 3.28 (1H, under the signal of the solvent), 5.09 (1H), 5.92 (1H), 6.55-6.72 (4H), 7.21-7.39 (2H), 7.95 (1H), 8.19 (1H), 8.33 (1H).

### Example 26

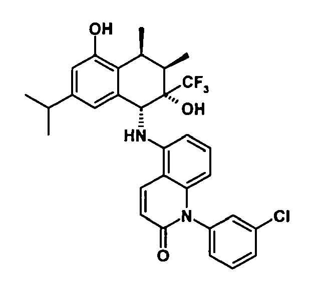

### (1RS, 2SR, 3RS, 4SR)- 5-{[2,5-Dihydroxy-7-isopropyl-3,4-dimethyl-2-(trifluoromethyl)-1,2,3,4-tetrahydronaphthalene-1-yl]amino}-1-(3-chlorophenyl)-1H-quinoline-2-one

5-{[2,5-Dihydroxy-7-isopropyl-3,4-dimethyl-2-(trifluoromethyl)-1,2,3,4-tetrahydronaphthalene-1-yl]amino}-1H-quinoline-2-one (160 mg, 0.35 mmol), described in example 23, 3-chlorophenyl-boronic acid (108.7 mg, 0.69 mmol), pyridine (0.56 mL, 6.95 mmol), copper(II)acetate (69.37 mg, 0.35 mmol) and 14.8 mL dichloromethane are stirred for three days at room temperature. The turquoise coloured solution is diluted with 150 mL ethyl acetate. The organic phase is washed twice with water (10 mL each) and brine (10 mL). After drying over sodium sulfate, the solvent is evaporated and the residue purified by chromatography (LiChroprep NH₂) eluting with dichloromethane/ methanol. The title compound is isolated with a yield of 11.4% (22.6 mg).

¹H-NMR (400 MHz, CD₃OD): δ = 1.08 (6H), 1.15 (3H), 1.42 (3H), 2.39 (1H), 2.62 (1H), 3.45 (1H), 5.10 (1H), 5.92 (1H), 6.53-6.63 (3H), 6.72 (1H), 7.22-7.30 (2H), 7.39 (1H), 7.55-7.65 (2H), 8.25 (1H).

### Example 27

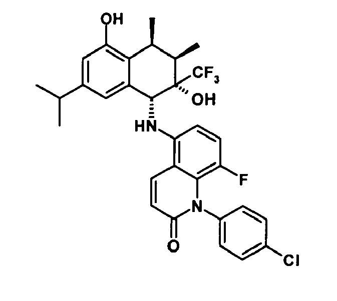

### (1RS, 2SR, 3RS, 4SR)-5-{[2,5-Dihydroxy-7-isopropyl-3,4-dimethyl-2-(trifluoromethyl)-1,2,3,4-tetrahydronaphthalene-1-yl]amino}-8-fluoro-1-(4-chlorophenyl)-1H-quinoline-2-one

5-{[2,5-Dihydroxy-7-isopropyl-3,4-dimethyl-2-(trifluoromethyl)-1,2,3,4-tetrahydronaphthalene-1-yl]amino}-1H-quinoline-2-one (80 mg, 0.17 mmol), 4-chlorophenyl-boronic acid (54.3 mg, 0.35 mmol), pyridine (0.28 mL, 3.47 mmol), copper(II)acetate (34.7 mg, 0.17 mmol) and 7.4 mL dichloromethane are stirred for three days at room temperature. The turquoise coloured solution is diluted with 50 mL ethyl acetate. The organic phase is washed twice with water (10 mL each) and brine (10 mL). After drying over sodium sulfate, the solvent is evaporated and the residue purified by chromatography (LiChroprep NH₂) eluting with dichloromethane/ methanol. 7.3 mg (7.4%) of the title compound are obtained. Further products are the O-arylated and the N,O-bisarylated compound.

¹H-NMR (300 MHz, CD₃OD): δ = 1.07 (6H), 1.14 (3H), 1.40 (3H), 2.39 (1H), 2.62 (1 H), 3.43 (1H), 4.99 (1H), 6.52-6.61 (3H), 6.69 (1H), 7.11 (1H), 7.25-7.35 (2H), 7.42-7.52 (2H), 8.19 (1H).

## Claims

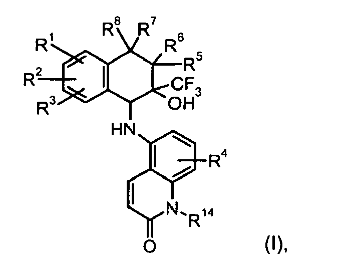

1. in which
R¹, R², R³ independently of one another, are selected from the group consisting of hydrogen, halogen, cyano, nitro, hydroxy, (C₁-C₅)-alkyl, (C₁-C₅)-halo-alkyl, (C₁-C₅)alkoxy, (C₁-C₅)halo-alkoxy and COOR¹³, and in which two vicinal substituents together may form a group that is selected from the groups -O-(CH₂)ₚ-O-, -O-(CH₂)ₚ-CH₂-, -O-CH=CH-, -(CH₂)ₚ₊₂-, -NH-(CH₂)ₚ₊₁-, -N(C₁-C₃-alkyl)-(CH₂)ₚ₊₁-, and -NH-N=CH-,
in which p = 1 or 2, and in which R¹³ means hydrogen or C₁-C₅-alkyl,
R⁴ is selected from the group consisting of hydrogen, halogen, cyano, nitro hydroxy, (C₁-C₅)-alkyl, (C₁-C₅)-halo-alkyl, (C₁-C₅)alkoxy, (C₁-C₅)halo-alkoxy and COOR¹³, in which R¹³ means hydrogen or C₁-C₅-alkyl,
R⁵, R⁶ independently of one another, are selected from the group consisting of hydrogen or (C₁-C₅)-alkyl or (C₁-C₅)-halo-alkyl,
R⁷, R⁸ independently of one another, are selected from the group consisting of hydrogen or (C₁-C₅)-alkyl, (C₁-C₅)-halo-alkyl, or in which R⁵ and R⁶ together, or R⁵ and R⁷ together, or R⁵ and R⁸ together, or R⁶ and R⁷ together, or R⁶ and R⁸ together, or R⁷ and R⁸ together may form a C₃-C₈ cycloalkyl ring,
R⁹, R¹⁰, R¹¹ independently of one another, are selected from the group consisting of hydrogen, halogen, cyano, nitro, hydroxy, (C₁-C₅)-alkyl, (C₁-C₅)-halo-alkyl, (C₁-C₅)alkoxy, (C₁-C₅)halo-alkoxy and COOR¹³, in which R¹³ means hydrogen or C₁-C₅-alkyl,
R¹⁴ is a group 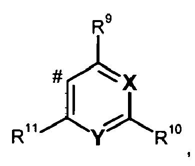 in which
# denotes the point of attachment of the R¹⁴ group via a single bond,
X, Y independently of one another are nitrogen, or a group C- R¹²
in which R¹² is selected from the group consisting of hydrogen, halogen, cyano, nitro, hydroxy, (C₁-C₅)-alkyl, (C₁-C₅)-halo-alkyl, (C₁-C₅)alkoxy, (C₁-C₅)halo-alkoxy and COOR¹³, in which R¹³ means hydrogen or C₁-C₅-alkyl.

2. One aspect of the invention are compounds of general formula I, in which
R¹, R², R³ independently of one another, are selected from the group consisting of hydrogen, halogen, hydroxy, (C₁-C₅)-alkyl, (C₁-C₅)alkoxy,
R⁴ is selected from the group consisting of hydrogen, halogen, hydroxy, or (C₁-C₅)-alkyl, (C₁-C₅)alkoxy,
R⁵, R⁶ independently of one another, are selected from the group consisting of hydrogen or (C₁-C₅)-alkyl,
R⁷, R⁸ independently of one another, are selected from the group consisting of hydrogen or (C₁-C₅)-alkyl, or in which R⁵ and R⁶ together, or R⁵ and R⁷ together, or R⁵ and R⁸ together, or R⁶ and R⁷ together, or R⁶ and R⁸ together, or R⁷ and R⁸ together may form a C₃-C₈ cycloalkyl ring,
R⁹, R¹⁰, R¹¹ independently of one another, are selected from the group consisting of hydrogen, halogen, cyano, hydroxy, (C₁-C₅)-alkyl, (C₁-C₅)-halo-alkyl,
R¹⁴ is a group 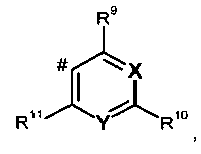 in which
# denotes the point of attachment of the R¹⁴ group via a single bond,
X, Y independently of one another are nitrogen, or a group C- R¹²
in which R¹² is selected from the group consisting of hydrogen, halogen, cyano, nitro, hydroxy, (C₁-C₅)-alkyl, (C₁-C₅)-halo-alkyl, (C₁-C₅)alkoxy, (C₁-C₅)halo-alkoxy and COOR¹³, in which R¹³ means hydrogen or C₁-C₅-alkyl.

3. Another aspect of the invention are compounds of general formula I, in which
R¹, R², R³ independently of one another, are selected from the group consisting of hydrogen, chloro, hydroxy, (C₁-C₄)-alkyl, methoxy,
R⁴ is selected from the group consisting of hydrogen or fluoro,
R⁵, R⁶ independently of one another, are selected from the group consisting of hydrogen or methyl,
R⁷, R⁸ independently of one another, are selected from the group consisting of hydrogen or methyl, or in which R⁵ and R⁶ together, or R⁵ and R⁷ together, or R⁵ and R⁸ together, or R⁶ and R⁷ together, or R⁶ and R⁸ together, or R⁷ and R⁸ together may form a C₃-C₈ cycloalkyl ring,
R⁹, R¹⁰, R¹¹ independently of one another, are selected from the group consisting of hydrogen, fluoro, chloro, cyano, hydroxy, methyl or trifluoromethyl,
R¹⁴ is a group 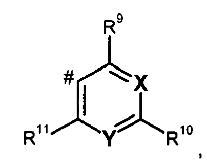 in which
# denotes the point of attachment of the R¹⁴ group via a single bond,
X, Y independently of one another are nitrogen or a group C- R¹²,
in which R¹² is selected from the group consisting of hydrogen, fluoro, chloro, cyano, hydroxyl or methyl.

4. compounds of general formula I chosen from a group consisting of: 5-{[2-Hydroxy-6-isopropyl-5-methoxy-4,4-dimethyl-2-(trifluoromethyl)-1,2,3,4-tetrahydronaphthalene-1-yl]amino}-1-(6-fluoropyridine-3-yl)-1H-quinoline-2-one
5-{[2,5-Dihydroxy-6-isopropyl-4,4-dimethyl-2-(trifluoromethyl)-1,2,3,4-tetrahydronaphthalene-1-yl]amino}-1-(6-fluoropyridine-3-yl)-1H-quinoline-2-one
5-{[6-Chloro-2,5-dihydroxy-4,4-dimethyl-2-(trifluoromethyl)-1,2,3,4-tetrahydronaphthalene-1-yl]amino}-1-(3-fluorophenyl)-1H-quinoline-2-one
5-{[2,5-Dihydroxy-4,4-dimethyl-2-(trifluoromethyl)-1,2,3,4-tetrahydronaphthalene-1-yl]amino}-1-(3-fluorophenyl)-1H-quinoline-2-one
5-{[6-Chloro-2,5-dihydroxy-4,4-dimethyl-2-(trifluoromethyl)-1,2,3,4-tetrahydronaphthalene-1-yl]amino}-1-(3-methylphenyl)-1H-quinoline-2-one
5-{[2,5-Dihydroxy-4,4-dimethyl-2-(trifluoromethyl)-1,2,3,4-tetrahydronaphthalene-1-yl]amino}-1-(3-methylphenyl)-1H-quinoline-2-one
5-{[6-Chloro-2,5-dihydroxy-4,4-dimethyl-2-(trifluoromethyl)-1,2,3,4-tetrahydronaphthalene-1-yl]amino}-1-(4-fluorophenyl)-1H-quinoline-2-one
5-{[2,5-Dihydroxy-4,4-dimethyl-2-(trifluoromethyl)-1,2,3,4-tetrahydronaphthalene-1-yl]amino}-1-(4-fluorophenyl)-1H-quinoline-2-one
5-{[6-Chloro-2,5-dihydroxy-4,4-dimethyl-2-(trifluoromethyl)-1,2,3,4-tetrahydronaphthalene-1-yl]amino}-1-(4-methylphenyl)-1H-quinoline-2-one
5-{[2,5-Dihydroxy-4,4-dimethyl-2-(trifluoromethyl)-1,2,3,4-tetrahydronaphthalene-1-yl]amino}-1-(4-methylphenyl)-1H-quinoline-2-one
5-{[6-Chloro-2-hydroxy-5-methoxy-4,4-dimethyl-2-(trifluoromethyl)-1,2,3,4-tetrahydronaphthalene-1-yl]amino}-1-(1,3-pyrimidine-5-yl)-1H-quinoline-2-one
5-{[6-Chloro-2,5-dihydroxy-4,4-dimethyl-2-(trifluoromethyl)-1,2,3,4-tetrahydronaphthalene-1-yl]amino}-1-(1,3-pyrimidine-5-yl)-1H-quinoline-2-one
5-{[6-Chloro-2,5-dihydroxy-4,4-dimethyl-2-(trifluoromethyl)-1,2,3,4-tetrahydronaphthalene-1-yl]amino}-1-(3-hydroxyphenyl)-1H-quinoline-2-one
5-{[6-Chloro-2,5-dihydroxy-4,4-dimethyl-2-(trifluoromethyl)-1,2,3,4-tetrahydronaphthalene-1-yl]amino}-1-(4-chlorophenyl)-1H-quinoline-2-one
5-{[2,5-Dihydroxy-4,4-dimethyl-2-(trifluoromethyl)-1,2,3,4-tetrahydronaphthalene-1-yl]amino}-1-(4-chlorophenyl)-1H-quinoline-2-one
5-{[6-Chloro-2,5-dihydroxy-4,4-dimethyl-2-(trifluoromethyl)-1,2,3,4-tetrahydronaphthalene-1-yl]amino}-1-(3-chlorophenyl)-1H-quinoline-2-one
5-{[2,5-Dihydroxy-4,4-dimethyl-2-(trifluoromethyl)-1,2,3,4-tetrahydronaphthalene-1-yl]amino}-1-(3-chlorophenyl)-1H-quinoline-2-one
5-{[6-Chloro-2-hydroxy-5-methoxy-4,4-dimethyl-2-(trifluoromethyl)-1,2,3,4-tetrahydronaphthalene-1-yl]amino}-1-(4-cyanophenyl)-1H-quinoline-2-one
5-{[6-Chloro-2,5-dihydroxy-4,4-dimethyl-2-(trifluoromethyl)-1,2,3,4-tetrahydronaphthalene-1-yl]amino}-1-(4-cyanophenyl)-1H-quinoline-2-one
5-{[6-Chloro-2-hydroxy-5-methoxy-4,4-dimethyl-2-(trifluoromethyl)-1,2,3,4-tetrahydronaphthalene-1-yl]amino}-1-(3-cyanophenyl)-1H-quinoline-2-one
5-{[6-Chloro-2,5-dihydroxy-4,4-dimethyl-2-(trifluoromethyl)-1,2,3,4-tetrahydronaphthalene-1-yl]amino}-1-(3-cyanophenyl)-1H-quinoline-2-one
5-{[6-Chloro-2,5-dihydroxy-4,4-dimethyl-2-(trifluoromethyl)-1,2,3,4-tetrahydronaphthalene-1-yl]amino}-1-(4-hydroxyhenyl)-1H-quinoline-2-one
*(1RS, 2SR, 3RS, 4SR)*-5-{[2,5-Dihydroxy-7-isopropyl-3,4-dimethyl-2-(trifluoromethyl)-1,2,3,4-tetrahydronaphthalene-1-yl]amino}-1-(6-fluoropyridin-3yl)-1H-quinoline-2-one
(1RS, 2SR, 3RS, 4SR)-5-{[2,5-Dihydroxy-7-isopropyl-3,4-dimethyl-2-(trifluoromethyl)-1,2,3,4-tetrahydronaphthalene-1-yl]amino}-8-fluoro-1-(6-fluoropyridin-3yl)-1H-quinoline-2-one
5-{[2,5-Dihydroxy-7-isopropyl-4-methyl-2-(trifluoromethyl)-1,2,3,4-tetrahydronaphthalene-1-yl]amino}-1-(6-fluoropyridin-3yl)-1H-quinoline-2-one
(1RS, 2SR, 3RS, 4SR)- 5-{[2,5-Dihydroxy-7-isopropyl-3,4-dimethyl-2-(trifluoromethyl)-1,2,3,4-tetrahydronaphthalene-1-yl]amino}-1-(3-chlorophenyl)-1H-quinoline-2-one
(1RS, 2SR, 3RS, 4SR)-5-{[2,5-Dihydroxy-7-isopropyl-3,4-dimethyl-2-(trifluoromethyl)-1,2,3,4-tetrahydronaphthalene-1-yl]amino}-8-fluoro-1-(4-chlorophenyl)-1H-quinoline-2-one

5. Process for preparing compounds of the formula I according to claim I, **characterized in that** quinoline derivatives of the formula II are reacted with boronic acid derivatives of the formula III

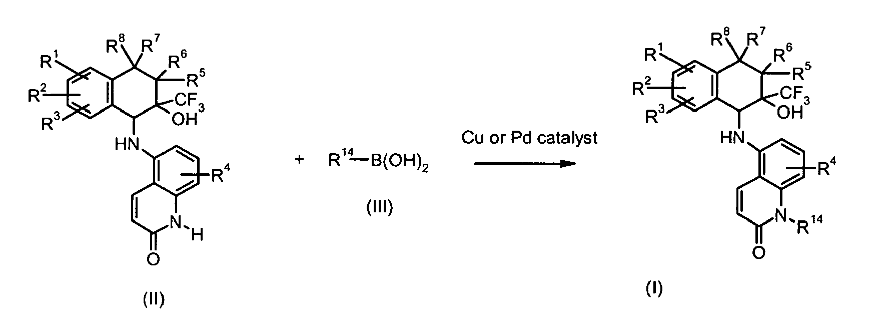

6. Process for preparing compounds of the formula I according to claim I, **characterized in that** an aldehyde of general formula IV, in which R¹, R², R³, R⁵, R⁶, R⁷ and R⁸ have the meanings that are indicated for formula I, and an aminoquinoline-2-one of general formula V, in which R⁴ and R¹⁴ are defined as described in formula I are reacted to form an imine which cyclises in the presence of a lewis acid.

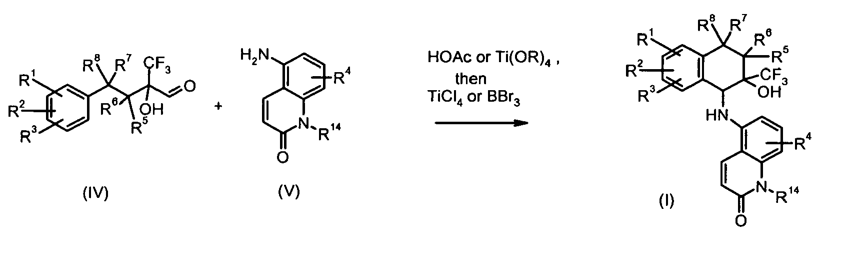

7. Use of the compounds according to at least one of claims 1-4 for the manufacture of pharmaceutical agents.

8. Use of the compounds according to at least one of claims 1-4 for the manufacture of pharmaceutical agents for treating inflammatory diseases.
